(19) Europäisches Patentamt
European Patent Office
Office européen des brevets

(11) EP 2 015 070 A1

(12) **EUROPEAN PATENT APPLICATION**
published in accordance with Art. 153(4) EPC

(43) Date of publication:
**14.01.2009 Bulletin 2009/03**

(21) Application number: **07742574.2**

(22) Date of filing: **20.04.2007**

(51) Int Cl.:
***G01N 33/53*** *(2006.01)* ***A61K 31/404*** *(2006.01)*
***A61P 35/00*** *(2006.01)* ***C12Q 1/02*** *(2006.01)*
***G01N 33/15*** *(2006.01)*

(86) International application number:
**PCT/JP2007/059139**

(87) International publication number:
**WO 2007/123274 (01.11.2007 Gazette 2007/44)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **20.04.2006 JP 2006117183**

(71) Applicant: **Eisai R&D Management Co., Ltd.**
**Tokyo 112-8088 (JP)**

(72) Inventor: **SEMBA, Taro**
**Tsukuba-shi, Ibaraki 300-2635 (JP)**

(74) Representative: **Woods, Geoffrey Corlett**
**J.A. KEMP & CO.**
**14 South Square**
**Gray's Inn**
**London**
**WC1R 5JJ (GB)**

# (54) NOVEL MARKER FOR SENSITIVITY AGAINST SULFONAMIDE COMPOUND

(57) The sensitivity of a tumor cell to a sulfonamide compound or the anti-tumor effect of a sulfonamide compound can be examined by determining the expression level of EGFR1 in the tumor cell and employing the variation in the EGFR1 expression level as a measure. Thus, a method for determination of the sensitivity of a tumor cell to a sulfonamide compound; a method for determination of the anti-tumor effect of a sulfonamide compound; and a test kit for use in these methods are provided.

EP 2 015 070 A1

## Description

FIELD OF THE INVENTION

The present invention relates to a method for examining the sensitivity of a tumor cell to a sulfonamide compound by examining change in the expression level of Epidermal Growth Factor Receptor 1 (also referred to as "EGF receptor 1" or "EGFR1") in the tumor cell under the action of a sulfonamide compound or an analog thereof, to a method for examining an anti-tumor effect of a sulfonamide compound, and to a test kit used for these methods.

BACKGROUND OF THE INVENTION

In conventional clinical trials for anti-tumor agents, the toxicity profile and the maximum recommended dose are firstly determined in phase I trial, followed by phase II trial in which the agents are assessed as drugs based on response rates using tumor regression as evaluation criteria. Meanwhile, along with the recent progress of cancer biology, drugs having novel mechanisms of inhibiting intracellular signal transuduction systems or angiogenesis (novel anticancer drugs) are going through active research and development. These novel anticancer drugs may not necessarily be administered at maximum recommended doses that come close to the toxic levels. Furthermore, improvements of QOL (Quality of Life) and life prolongation resulting from inhibition of tumor growth, rather than tumor regression, seem to be more appropriate criteria for judgement of the effects of the drugs. In this case, in order to verify the effects of the drugs in more logical and specific manner, the change in biological makers closely related to inhibition of tumor growth is desirably used as a surrogate marker.

Generally, in an anticancer therapy, responsiveness of an organism upon administration of an anticancer drug largely depends on the sensitivity of the target tumor cell to the drug. The sensitivity of a target tumor cell to a drug greatly varies by the respective tumor cells. The difference in the sensitivity results from quantitative or qualitative difference of the molecules targeted by the drugs or their relative factors, or acquirement of drug resistance or else. Given this background, if change specific to the target tumor cell showing sensitivity to the drug can be determined with a tumor tissue or the like obtained by biopsy or the like, this can be very beneficial as a surrogate marker which allows early evaluation of drug efficacy, establishment of treatment approach, choice of new treatment approach and the like. Furthermore, a tumor cell can be separated from a tumor tissue obtained before the treatment by biopsy or the like according to a conventional technique so that it can be treated with a drug to judge whether this tumor cell is sensitive to the drug by the change in the biological marker mentioned above, which is clinically helpful in that efficacy of treatment with said drug can be predicted in advance. It is critical that the change in this surrogate marker is specific to an anti-tumor effect and measurable in a highly sensitive manner. Specifically, a surrogate marker may be any one of quantitative determination of alteration of gene expression specific to the anti-tumor effect of the drug, an analysis of quantitative alteration of protein having the change in gene expressions, and an analysis of functional alteration associated with the change.

Recently, a sulfonamide compound has been reported as a useful anti-tumor agent (Documents 1-5). In particular,
N-(3-chloro-1H-indole-7-yl)-1,4-benzenedisulfonamide (hereinafter, also referred to as "E7070"), N-(3-cyano-4-methyl-1H-indole-7-yl)-3-cyanobenzenesulfonamide (hereinafter, also referred to as "E7820"),
N-[[(4-chlorophenyl)amino]carbonyl]-2,3-dihydro-1H-indene-5-sulfonamide (hereinafter, also referred to as "LY186641"),
N-[[(3,4-dichlorophenyl)amino]carbonyl]-2,3-dihydrobenzofuran-5-sulfonamide (hereinafter, also referred to as "LY29550.1"),
N-(2,4-dichlorobenzoyl)-4-chlorophenylsulfonamide (hereinafter, also referred to as "LY-ASAP"), N-(2,4-dichlorobenzoyl)-5-bromothiophene-2-sulfonamide (hereinafter, also referred to as "LY573636") and 2-sulfanylamide-5-chloroquinoxaline (hereinafter, also referred to as "CQS") are active against various types of tumors and thus are highly useful.

The sulfonamide compounds mentioned above have also been reported to show identical or similar actions and effects (Documents 6-8).

For early establishment of clinical therapy through rapid clinical development of the useful anti-tumor drugs mentioned above and for contribution to the improvement of QOL of the patients through efficient treatment based on the established therapy, it is desirable to fmd and apply specific surrogate markers that can be specifically employed upon administration of the anti-tumor drugs.

In recent years, surrogate markers relative to the sulfonamide compounds have been reported (Documents 8 and 9). Specifically, changes in gene expressions described in WO02/42493 and WO2006/036025 have been reported useful as surrogate markers for anti-tumor effects of the sulfonamide compounds.

References

(1) Japanese Laid-Open Patent Publication No. 7-165708
(2) International Publication No. WO00/50395
(3) European Patent Publication No. 0222475
(4) International Publication No. WO02/098848
(5) International Publication No. WO03/035629
(6) International Publication No. WO2006/030941
(7) International Publication No. WO2006/030947
(8) International Publication No. WO2006/036025
(9) International Publication No. WO02/042493

DISCLOSURE OF THE INVENTION

The present invention was achieved regarding the circumstances described above. The problems to be solved by the invention are to find a protein whose expression level changes when a sulfonamide compound having anti-tumor activity acts on a tumor cell, and to use this protein as a surrogate marker for the anti-tumor effect of the sulfonamide compound.

In order to solve the above problem, the present inventors have gone through keen examination and studied the changes in expression levels of EGFR1 upon treatment with E7820 for cancer cell lines highly sensitive to E7820 (PC9 and HCT116) and cancer cell lines less sensitive to E7820 (A549 and WiDr). Whereas the EGFR1 expression level and the EGFR1 phosphorylation level decreased for the cancer cell lines highly sensitive to E7820, the EGFR1 expression level showed barely any change for the cancer cell lines less sensitive to E7820. These results prove that EGFR1 expression level can be used as a surrogate marker for E7820 action.

In addition, change in the EGFR1 expression level after E7070 treatment was studied for cancer cell line highly sensitive to E7070 (HCT116-C9 (Molecular Cancer Therapeutics, 2002, 1, 275-286)), where the EGFR1 expression level was decreased by E7070.

According to experiments using DNA microarrays and cancer cell line panels, genetic alteration patterns were found to show high correlation with antiproliferative activities, for E7070, E7820, LY186641, LY295501, LY573636, CQS or combinations thereof (WO2006/030941, WO2006/030947 and WO2006/036025). In an assay for determining an antiproliferative activity, a cancer cell line resistant to E7070 was found to show cross-resistance to E7820, LY186641, LY295501, LY-ASAP, LY573636 or CQS (WO2006/030941 and WO2006/030947). E7070, E7820, LY186641, LY295501, LY-ASAP, LY573636, CQS or combinations thereof have been reported to give identical or similar action mechanisms that result in identical or similar actions and effects (WO2006/030941, WO2006/030947 and WO2006/036025).

Accordingly, we found that the sensitivity of a tumor cell to a sulfonamide compound, preferably E7070, E7820, LY186641, LY295501, LY-ASAP, LY573636, CQS or a combination thereof, allows judgment that a patient is highly sensitive to the sulfonamide compound when the EGFR1 expression level in a tumor cell removed from the cancer patient after the administration of the sulfonamide compound is decreased compared to the EGFR1 expression level in a tumor cell removed from the cancer patient before the administration of the sulfonamide compound and/or when the EGFR1 expression level in a tumor cell removed from a cancer patient and treated with the sulfonamide compound is decreased compared to the EGFR1 expression level in an untreated tumor cell.

We also found that a sulfonamide.compound, preferably E7070, E7820, LY186641, LY295501, LY-ASAP, LY573636, CQS or a combination thereof can be judged to be exerting an anti-tumor effect if the EGFR1 expression level in a tumor cell removed from a cancer patient after the administration of the sulfonamide compound is decreased compared to the EGFR1 expression level in a tumor cell removed from the cancer patient before the administration of the sulfonamide compound.

Thus, the present invention relates to the followings.

(1) A method for examining the sensitivity of a tumor cell to a sulfonamide compound or an analog thereof, comprising the steps of:

(a) determining the EGFR1 expression level in a tumor cell removed from a cancer patient before and after the administration of the sulfonamide compound or the analog thereof; and
(b) judging that the tumor cell is highly sensitive to the sulfonamide compound or the analog thereof if the EGFR1 expression level in the tumor cell removed after the administration of the sulfonamide compound or the analog

thereof is decreased compared to the EGFR1 expression level in the tumor cell removed before the administration of said compound.

(2) A method for examining the sensitivity of a tumor cell to a sulfonamide compound or an analog thereof, comprising the steps of:

(a) treating a tumor cell removed from a cancer patient with the sulfonamide compound or the analog thereof;
(b) determining the EGFR1 expression levels in the tumor cell treated in step (a) and an untreated tumor cell; and
(c) judging that the tumor cell is highly sensitive to the sulfonamide compound or the analog thereof if the EGFR1 expression level in the tumor cell treated in step (a) is decreased compared to the EGFR1 expression level in the untreated tumor cell.

(3) A method for examining the sensitivity of a tumor cell to a sulfonamide compound or an analog thereof, comprising the steps of:

(a) determining the EGFR1 expression level in a tumor cell of a cancer patient before and after the administration of the sulfonamide compound or the analog thereof; and

(b) judging that the tumor cell is highly sensitive to the sulfonamide compound or the analog thereof if the EGFR1 expression level in the tumor cell after the administration of the sulfonamide compound or the analog thereof is decreased compared to the EGFR1 expression level in the tumor cell before the administration of said compound.

(4) A method for examining the sensitivity of a tumor cell to a sulfonamide compound or an analog thereof, comprising the steps of:

(a) determining the expression level of EGFR1 in soluble form in blood drawn from a cancer patient before and after the administration of the sulfonamide compound or the analog thereof; and
(b) judging that the tumor cell is highly sensitive to the sulfonamide compound or the analog thereof if the expression level of EGFR1 in soluble form in blood drawn after the administration of the sulfonamide compound or the analog thereof is decreased compared to the expression level of EGFR1 in soluble form in blood drawn before the administration of said compound.

(5) A method for examining an anti-tumor effect of a sulfonamide compound or an analog thereof, comprising the steps of:

(a) determining the EGFR1 expression level in a tumor cell removed from a cancer patient before and after the administration of the sulfonamide compound or the analog thereof; and
(b) judging that the sulfonamide compound or the analog thereof is exerting an anti-tumor effect if the EGFR1 expression level in the tumor cell removed after the administration of the sulfonamide compound or the analog thereof is decreased compared to the EGFR1 expression level in the tumor cell removed before the administration of said compound.

(6) A method for examining an anti-tumor effect of a sulfonamide compound or an analog thereof, comprising the steps of:

(a) determining the EGFR1 expression level in a tumor cell of a cancer patient before and after the administration of the sulfonamide compound or the analog thereof; and

(b) judging that the sulfonamide compound or the analog thereof is exerting an anti-tumor effect if the EGFR1 expression level in the tumor cell after the administration of the sulfonamide compound or the analog thereof is decreased compared to the EGFR1 expression level in the tumor cell before the administration of said compound.

(7) A method for examining an anti-tumor effect of a sulfonamide compound or an analog thereof, comprising the steps of:

(a) determining the expression level of EGFR1 in soluble form in blood drawn from a cancer patient before and

after the administration of the sulfonamide compound or the analog thereof; and
(b) judging that the tumore cell or the sulfonamide compound or the analog thereof is exerting an anti-tumor effect if the expression level of EGFR1 in soluble form in blood drawn after the administration of the sulfonamide compound or the analog thereof is decreased compared to the expression level of EGFR1 in soluble form in blood drawn before the administration of said compound.

The sulfonamide compounds or analogs thereof according to (1)-(7) above include at least one compound selected from the group consisting of:

a compound represented by General Formula (I)

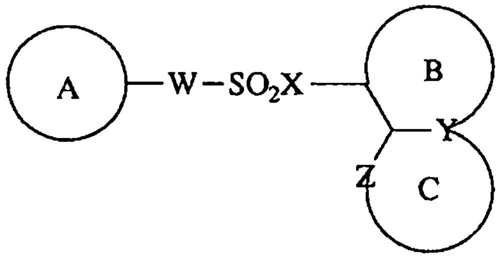

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents a carbon atom or a nitrogen atom, and
Z represents $-N(R^2)-$ or a nitrogen atom,
wherein, $R^1$ and $R^2$ each independently represent, identically or differently, a hydrogen atom or a lower alkyl group];
a compound represented by General Formula (II)

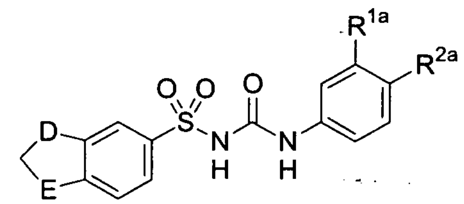

(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

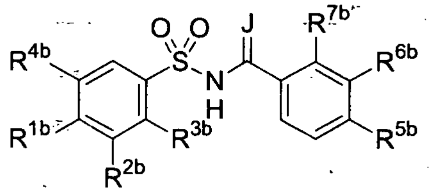

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, -O

$(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-C6 alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either one of $R^{5b}$ or $R^{7b}$ is an optionally substituted $C_1$-$C_6$ alkyl group or when $R^{7b}$ is a halogen atom or an optionally substituted $C_1$-$C_6$ alkyl group, either one of $R^{5b}$ or $R^{6b}$ is a hydrogen atom)];

a compound represented by Formula (IV)

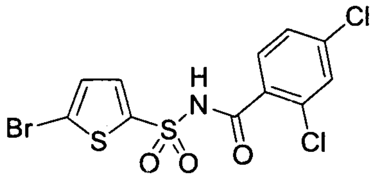

(IV)

;

and

a compound represented by Formula (V)

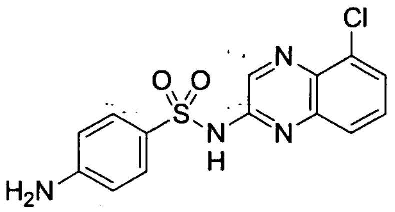

(V)

,

or a pharmacologically acceptable salt thereof, or a solvate thereof.

The present invention also relates to the followings.

(8) A method according to any one of (1) to (7) above, wherein the sulfonamide compound is at least one compound selected from the group consisting of:

N-(3-chlor6-1H-mdole-7-yl)-1,4-benzenedisulfonamide

N-(3-cyano-4-methyl-1H-indole-7-yl)-3-cyanobenzenesulfonamide,

N-[[(4-chlorophenyl)amino]carbonyl]-2,3-dihydro-1H-indene-5-sulfonamide,

N-[[(3,4-dichlorophenyl)amino]carbonyl]-2,3-dihydrobenzofuran-5-sulfonamide,

N-(2,4-dichlorobenzoyl)-4-chlorophenylsulfonamide,

N-(2,4-dichlorobenzoyl)-5-bromothiophene-2-sulfonamide and

2-sulfanylamide-5-chloroquinoxaline,

a pharmacologically acceptable salt thereof or a solvate thereof.

(9) A method according to any one of (1) to (7), wherein the sulfonamide compound is N-(3-chloro-1H-indole-7-yl)-1,4-benzenedisulfonamide, a pharmacologically acceptable salt thereof or a solvate thereof.

(10) A method according to any one of (1) to (7), wherein the sulfonamide compound is N-(3-cyano-4-methyl-1H-indole-7-yl)-3-cyanobenzenesulfonamide, a pharmacologically acceptable salt thereof or a solvate thereof.

(11) A method according to any one of (1) to (7), wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, a pharmacologically acceptable salt thereof or a solvate thereof.

(12) A method according to any one of (1) to (7), wherein the sulfonamide compound is N-(2,4-dichlorobenzoyl)-5-bromothiophene-2-sulfonamide in sodium salt form.
(13) A method according to any one of (1) to (12), wherein the determination of the EGFR1 expression level is carried out by quantifying protein of EGFR1.
(14) A method according to (13), wherein the quantification of protein is carried out by at least one selected from the group consisting of immunochemical technique, mass spectroscopy, SPECT and PET.
(15) A method according to (14), wherein the immunochemical technique is at least one technique selected from the group consisting of immunohistochemical technique, flow cytometry and western blotting.
(16) A test kit used with the method according to (13), comprising an antibody having affinity for EGFR1.
(17) A method according to any one of (1) to (12), wherein the determination of the EGFR1 expression level is carried out by quantifying the phosphorylation level of EGFR1.
(18) A method according to (17), wherein the quantification of the phosphorylation level of EGFR1 is carried out by at least one selected from the group consisting of immunochemical technique, mass spectroscopy, SPECT and PET.
(19) A method according to (18), wherein the immunochemical technique is at least one technique selected from the group consisting of immunohistochemical technique, flow cytometry and western blotting.
(20) A test kit used with the method according to (17), comprising an antibody having affinity for phosphorylated EGFR1.

Neither International Publication No. WO02/42493 nor International Publication No. WO2006/036025 describe that the sensitivity of a tumor cell to a sulfonamide compound can be examined by examining the change in the EGFR1 expression level in a tumor cell removed from a cancer patient who has been administered with the sulfonamide compound. Moreover, decrease in the EGFR1 expression level by the sulfonamide compound has been unfound despite the exhaustive examinations using DNA microarrays for genes that change in expression levels in tumor cells by sulfonamide compounds, which proves that this is hardly predictable by those skilled in the art.

The present invention allows examination of the sensitivity of tumor cells to sulfonamide compounds.

More specifically, the present invention allows examination of the sensitivity of a tumor cell to a sulfonamide compound, preferably E7070, E7820, LY186641, LY295501, LY-ASAP, LY573636, CQS or a combination thereof, using change in the EGFR1 expression level as a measure where the EGFR1 expression level in a tumor cell removed from a cancer patient after the administration of the sulfonamide compound is compared with that before the administration of the sulfonamide compound, and/or change in the EGFR1 expression level as a measure where the EGFR1 expression level in a tumor cell removed from a cancer patient and treated with the sulfonamide compound is compared with the EGFR1 expression level in an untreated tumor cell.

Examination of the sensitivity of a tumor cell to a sulfonamide compound is highly useful in that cancer patients can be selected who could be prospective candidates for obtaining higher anti-tumor effects in clinical practice.

In addition, the present invention allows examination of a sulfonamide compound for an anti-tumor effect.

More specifically, the present invention allows examination of a sulfonamide compound, preferably E7070, E7820, LY186641, LY295501, LY-ASAP, LY573636, CQS or a combination thereof, for its anti-tumor effect by using change in the EGFR1 expression level as a measure where EGFR1 expression level in the tumor cell removed from a cancer patient after the administration of the sulfonamide compound is compared with that before the administration of the sulfonamide compound.

Examination of a sulfonamide compound for an anti-tumor effect is remarkably useful in determining suitable dosage or the like in clinical practice.

BRIEF DESCRIPTION OF THE DRAWINGS

Figure 1 shows an anti-tumor effect of E7820 on a subcutaneous transplant model of human non-small cell lung cancer cell line (PC9).
Figure 2 shows an anti-tumor effect of E7820 on a subcutaneous transplant model of human non-small cell lung cancer cell line (A549).
Figure 3 shows an anti-tumor effect of E7820 on a subcutaneous transplant model of human colon cancer cell line (WiDr).
Figure 4 shows an anti-tumor effect of E7820 on a subcutaneous transplant model of human colon cancer cell line (HCT116).
Figure 5 is a photograph of immunohistologic stains indicating an anti-tumor effect of E7820 on a subcutaneous transplant model of human non-small cell lung cancer cell line (PC9). Stains (a) to (f) show stain with control IgG (negative control), E7820-unadministered group, E7820-administered group (25 mg/kg), E7820-administered group

(50 mg/kg), E7820-administered group (100 mg/kg) and E7820-administered group (200 mg/kg), respectively.

BEST MODES FOR CARRYING OUT THE INVENTION

Hereinafter, embodiments of the present invention will be described. The following embodiments are described for illustrating the present invention and they are not intended to limit the present invention. The present invention may be carried out in various embodiments without departing from the scope of the invention.

The publications, laid-open patent publications, patent publications and other patent documents cited herein are incorporated herein by reference. The present specification also incorporates the disclosure of Japanese Patent Application No. 2006-117183 based on which the present application claims priority.

1. Sulfonamide compound

According to the present invention, a sulfonamide compound or an analog thereof (hereinafter, simply referred to as a "sulfonamide compound") comprises a compound represented by the following General Formula (I).

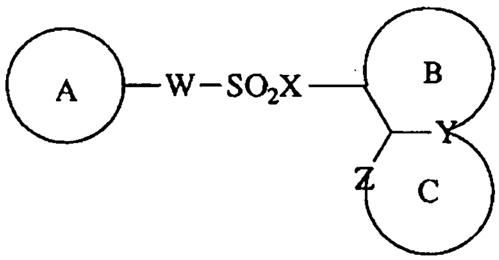

(I)

In General Formula (I),

ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents -N($R^1$)- or an oxygen atom,
Y represents a carbon atom or a nitrogen atom,
Z represents -N($R^2$)- or a nitrogen atom.

Herein, $R^1$ and $R^2$ each independently represent, identically or differently, a hydrogen atom or a lower alkyl group.

Herein, "an analog of a sulfonamide compound" refers to a compound where X is an oxygen atom in the compound represented by Formula (I).

In General Formula (I) above, "an optionally substituted monocyclic or bicyclic aromatic ring" meant by ring A is an aromatic hydrocarbon or an aromatic heterocycle containing at least one of a nitrogen atom, an oxygen atom and a sulfur atom, which may have 1 to 3 substituents on the ring. Examples of the aromatic ring comprised in ring A mainly include pyrrole, pyrazole, imidazole, thiophene, furan, thiazole, oxazole, benzene, pyridine, pyrimidine, pyrazine, pyridazine, naphthalene, quinoline, isoquinoline, phthalazine, naphthyridine, quinoxaline, quinazoline, cinnoline, indole, isoindole, indolizine, indazole, benzofuran, benzothiophene, benzoxazole, benzimidazole, benzopyrazole and benzothiazole, although the aromatic ring comprised in ring A is not limited thereto. The aromatic ring may have 1 to 3 substituents, and when more than one substituent exist, they may be identical or different. Examples of the substituent include an amino group that may be substituted with a lower alkyl group or a lower cyclo alkyl group, a lower alkyl group, a lower alkoxy group, a hydroxyl group, a nitro group, a mercapto group, a cyano group, a lower alkylthio group, a halogen atom, a group represented by Formula -a-b [wherein, a represents a single bond, $-(CH_2)_k-$, $-O-(CH_2)_k-$, $-S-(CH_2)_k-$ or $-N(R^3)-(CH_2)_k-$, k represents an integer of 1-5, $R^3$ refers to a hydrogen atom or a lower alkyl group, b represents $-CH_2-d$ (wherein, d represents amino group that may be substituted with a lower alkyl group, a halogen atom, a hydroxyl group, a lower alkylthio group, a cyano group or a lower alkoxy group)], a group represented by Formula -a-e-f [wherein, a is as stated above, e represents -S(O)- or $-S(O)_2-$, f represents an amino group that may be substituted with a lower alkyl group or a lower alkoxy group, a lower alkyl group, a trifluoromethyl group, $-(CH_2)_m-b$ or $-N(R^4)-(CH_2)_m-b$ (wherein, b is as stated above, $R^4$ represents a hydrogen atom or a lower alkyl group, m represents an integer of 1-5)], a group

represented by Formula -a-g-h [wherein, a is as stated above, g represents -C(O)- or -C(S)-, h represents an amino group that may be substituted with a lower alkyl group, a hydroxyl group, a lower alkyl group, a lower alkoxy group, $-(CH_2)_n$-b or $-N(R^5)-(CH_2)_n$-b (wherein, b is as stated above, $R^5$ represents a hydrogen atom or a lower alkyl group, and n represents an integer of 1-5)], a group represented by Formula $-a-N(R^6)$-g-i [wherein, a and g are as stated above, $R^6$ represents a hydrogen atom or a lower alkyl group, and i represents a hydrogen atom, a lower alkoxy group or f (f is as stated above)], a group represented by Formula $-a-N(R^7)$-e-f (wherein, a, e and f are as stated above, $R^7$ refers to a hydrogen atom or a lower alkyl group), and a group represented by Formula $-(CH_2)_p-j-(CH_2)_q$-b (wherein, j represents an oxygen atom or a sulfur atom, b is as stated above, p and q identically or differently represent an integer of 1-5).

For the exemplary substituents mentioned above, when the amino group is substituted with two alkyl groups, these alkyl groups may bind to each other to form a 5 or 6-membered ring. When ring A is a nitrogen-containing heterocycle containing a hydroxyl group or a mercapto group, these groups may take a resonance structure and form an oxo group or a thioxo group.

In General Formula (I), "an optionally substituted 6-membered cyclic unsaturated hydrocarbon or 6-membered unsaturated heterocycle containing a nitrogen atom as a heteroatom" meant by ring B, for example, is benzene or pyridine in which a part of the unsaturated binding may be hydrogenated, which may have one or two substituents on the ring. When two or more substituents exist, they may be identical or different.

"An optionally substituted 5-membered heterocycle containing one or two nitrogen atoms" meant by ring C is pyrrole, pyrazole or imidazole in which a part of the unsaturated binding may be hydrogenated, which may have one or two substituents on the ring. When two substituents exist, they may be identical or different.

Examples of substituents that rings B and C may have include but not limited to a halogen atom, a cyano group, a lower alkyl group, a lower alkoxy group, a hydroxyl group, an oxo group, Formula -C(O)-r (wherein, r represents a hydrogen atom, an amino group that may be substituted with a lower alkyl group, a lower alkyl group, a lower alkoxy group or a hydroxyl group), an amino group that may be substituted with a lower alkyl group and a trifluoromethyl group.

In General Formula (I), a "lower alkyl group" in the definition of $R^1$ and $R^2$ and the definition of the substituents that ring A, ring B and ring C may have refers to a linear or branched alkyl group with a carbon number of 1-6, for example, but not limited to, a methyl group, an ethyl group, a n-propyl group, an isopropyl group, a n-butyl group, an isobutyl group, a sec-butyl group, a tert-butyl group, a n-pentyl group (an amyl group), an isopentyl group, a neopentyl group, a tert-pentyl group, a 1-methylbutyl group, a 2-methylbutyl group, a 1,2-dimethylpropyl group, a n-hexyl group, an isohexyl group, a 1-methylpentyl group, a 2-methylpentyl group, a 3-methylpentyl group, a 1-ethylpropyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group and a 1-ethyl-2-methylpropyl group. Among these, examples of preferable groups include a methyl group, an ethyl group, a n-propyl group, an isopropyl group, a n-butyl group, an isobutyl group, a sec-butyl group and a tert-butyl group, while examples of more preferable groups include a methyl group, an ethyl group, a n-propyl group and an isopropyl group, the most preferably group being an ethyl group.

The "lower cyclo alkyl group" in the definition of the substituents that ring A may have refers to a cyclo alkyl group with a carbon number of 3-8, for example, but not limited to, a cyclopropyl group, a cyclobutyl group, a cyclopentyl group, a cyclohexyl group, a cycloheptyl group and a cyclooctyl group. The "lower alkylthio group" also refers to an alkylthio group derived from the lower alkyl group, for example, but not limited to, a methylthio group, an ethylthio group, a n-propylthio group, an isopropylthio group, a n-butylthio group, an isobutylthio group, a sec-butylthio group and a tert-butylthio group.

The "lower alkoxy group" in the definition of the substituents that ring A, ring B and ring C may have, for example, refers to, but not limited to, a lower alkoxy group derived from a lower alkyl group mentioned above such as a methoxy group, an ethoxy group, a n-propoxy group, an isopropoxy group, a n-butoxy group, an isobutoxy group, a sec-butoxy group and a tert-butoxy group, the most preferable group being a methoxy group and an ethoxy group. In addition, examples of a "halogen atom" include a fluorine atom, a chlorine atom, a bromine atom and an iodine atom.

The compound represented by General Formula (I) of the invention can be produced according to a known method, for example, by those described in International Publication No. 95/07276 (WO95/07276) and/or Japanese Laid-Open Patent Publication No. 7-165708 (JP7-165708).

In General Formula (I), a preferable compound is E7070 or E7820.

E7070 is N-(3-chloro-1H-indole-7-yl)-1,4-benzenedisulfonamide, whose structural formula is represented by the following Formula (VI).

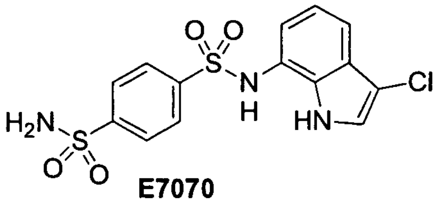

(VI)

E7070

E7070 can be produced according to a known method, for example, by those described in International Publication No. 95/07276 (WO95/07276) and/or Example 19 of Japanese Laid-Open Patent Publication No. 7-165708 (JP7-165708).

E7070 may also be referred to as N-(3-chloro-1H-indole-7-yl)-4-sulfamoylbezensulfonamide.

E7820 is N-(3-cyano-4-methyl-1H-indole-7-yl)-3-cyanobenzenesulfonamide, whose structural formula is represented by the following Formula (VII).

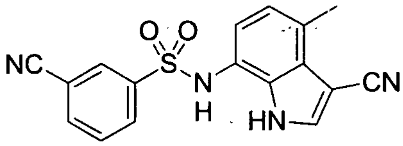

(VII)

E7820

E7820 can be produced according to a known method, for example, by a method described in International Publication No. 00/50395 (WO00/50395). E7820 may also be referred to as 3-cyano-N-(3-cyano-4-methyl-1H-indole-7-yl)benzenesulfonamide.

According to the present invention, the sulfonamide compound comprises a compound represented by the following General Formula (II).

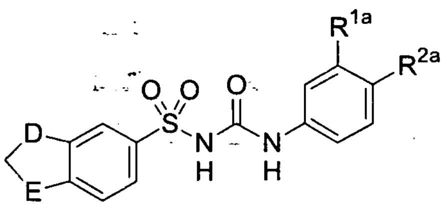

(II)

In General Formula (II) above, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom (e.g., a fluorine atom, a chlorine atom, a bromine atom or an iodine atom), and $R^{2a}$ represents a halogen atom or a trifluoromethyl group.

The compound represented by General Formula (II) of the invention can be produced according to a known method, for example, by a method described in European Patent Publication No. 0222475A1 (EP0222475A1).

In General Formula (II), a preferable compound is LY186641 or LY295501.

LY186641 is N-[[(4-chlorophenyl)amino]carbonyl]-2,3-dihydro-1H-indene-5-sulfonamide, whose structural formula is represented by the following Formula (VIII).

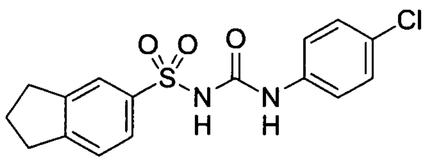

(VIII)

LY186641

LY186641 can be produced according to a known method, for example, by a method described in European

Patent Publication No. 0222475A1 (EP0222475A1).

According to the present invention, LY295501 is N-[[(3,4-dichlorophenyl)amino]carbonyl]-2,3-dihydrobenzofuran-5-sulfonamide, whose structural formula is represented by the following Formula (IX).

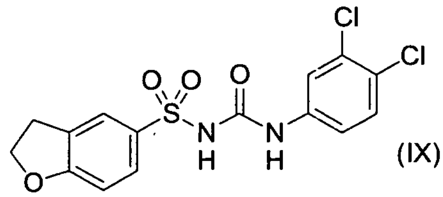

(IX)

LY295501

LY295501 can be produced according to a known method, for example, by those described in European Patent Publication No. 0222475A1 (EP0222475A1) and/or European Patent Publication No. 0555036A2 (EP0555036A2).

Furthermore, according to the present invention, the sulfonamide compound comprises a compound represented by the following General Formula (III).

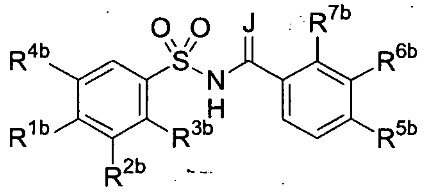

(III)

In General Formula (III), J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ refers to a hydrogen atom, a halogen atom, an optionally substituted $C_1$-$C_6$ alkyl group, $-CF_3$ or a nitro group, $R^{6b}$ refers to a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ refers to a halogen atom, an optionally substituted $C_1$-$C_6$ alkyl group or $-CF_3$ (provided that when either $R^{5b}$ or $R^{7b}$ is an optionally substituted $C_1$-$C_6$ alkyl group or when $R^{7b}$ is a halogen atom or an optionally substituted $C_1$-$C_6$ alkyl group, either $R^{5b}$ or $R^{6b}$ is a hydrogen atom).

In General Formula (III), a "halogen atom" is preferably a fluorine atom, a chlorine atom, a bromine atom or an iodine atom.

In General Formula (III), "$C_1$-$C_6$ alkyl group" is synonymous with the "lower alkyl group" described above, and preferably includes, but not limited to, a methyl group, an ethyl group, a n-propyl group, an isopropyl group, a n-butyl group, an isobutyl group, a sec-butyl group, a tert-butyl group, a n-pentyl group and a n-hexyl group.

In General Formula (III), "$C_1$-$C_4$ alkoxy group" refers to an alkoxy group with a carbon number of 1-4 of the "lower alkoxy groups" described above, and preferably includes, but not limited to, a methoxy group, an ethoxy group, a n-propoxy group, an isopropoxy group, a n-butoxy group, an isobutoxy group, a sec-butoxy group and a tert-butoxy group.

In General Formula (III), examples of alkyl group of "$C_1$-$C_4$ alkylthio group" include, but not limited to, a methyl group, an ethyl group, a propyl group an isopropyl group, a n-butyl group, an isobutyl group, a sec-butyl group and a tert-butyl group.

In General Formula (III), examples of "$C_1$-$C_4$ alkoxy carbonyl group" include, but not limited to, a methoxy carbonyl group, an ethoxy carbonyl group, a n-propoxy carbonyl group, an isopropoxy carbonyl group, a n-butoxy carbonyl group, an isobutoxy carbonyl group, a sec-butoxy carbonyl group and a tert-butoxy carbonyl group.

In General Formula (III), examples of substituents to be introduced include, but not limited to, substituents such as a $C_1$-$C_6$ alkyl group (e.g., a methyl group, an ethyl group, a n-propyl group, an isopropyl group, a n-butyl group, an isobutyl group, a sec-butyl group, a tert-butyl group, etc.), a $C_1$-$C_4$ alkoxy group (e.g., a methoxy group, an ethoxy group, a n-propoxy group, an isopropoxy group, a n-butoxy group, an isobutoxy group, a sec-butoxy group, a tert-butoxy group, etc.), an amino group, a hydroxyl group, a halogen atom (e.g., a fluorine atom, a chlorine atom, a bromine atom or an iodine atom) and a silyl group.

The compound represented by General Formula (III) of the invention can be produced by a known method such as the method described in International Publication No. 02/098848 (WO02/098848).

In General Formula (III), a preferable compound is LY-ASAP.

LY-ASAP is N-(2,4-dichlorobenzoyl)-4-chlorophenylsulfonamide, whose structural formula is represented by the following Formula (X).

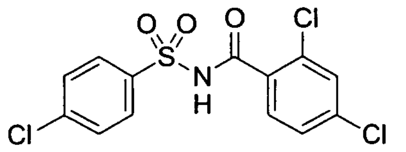

(X)

**LY-ASAP**

LY-ASAP can be produced by a known method such as the method described in International Publication No. 02/098848 (WO02/098848).

According to the present invention, an example of the sulfonamide compound further includes LY573636. According to the invention, LY573636 is N-(2,4-dichlorobenzoyl)-5-bromothiophene-2-sulfonamide, whose structural formula is represented by the following Formula (IV).

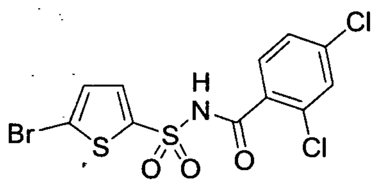

(IV)

LY573636

LY573636 is preferably in sodium salt form.

LY573636 can be produced by a known method. For example, it can be produced in the same manner as the method described in International Publication No. 02/098848 (WO02/098848) using commercially available 5-bromothiophene-2-sulfonyl chloride and 2,4-dichlorobenzoic acid.

LY573636 can also be produced by a method described in Example 63 of International Publication No. 2003/035629 (WO2003/035629).

According to the present invention, the sulfonamide compound may be CQS. According to the present invention, CQS is 2-sulfanylamide-5-chloroquinoxaline, whose structural formula is represented by the following Formula (V).

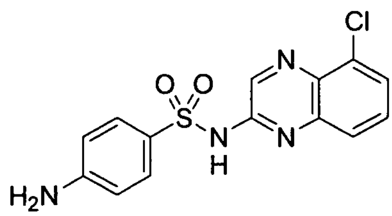

(V)

**CQS**

CQS can be produced according to a known method, for example, by a method described in J. Am. Chem. Soc., 1947, 71, 6-10.

The sulfonamide compound may form a pharmacologically acceptable salt with acid or base. The sulfonamide compound of the invention also comprises these pharmacologically acceptable salts. Examples of salts formed with acids include inorganic acid salts such as hydrochloride salts, hydrobromide salts, sulfate salts and phosphate salts, and salts formed with organic acids such as formic acid, acetic acid, lactic acid, succinic acid, fumaric acid, maleic acid, citric acid, tartaric acid, benzoic acid, methanesulfonic acid, benzenesulfonic acid, p-toluenesulfonic acid and trifluoroacetic acid. Examples of salts formed with bases include alkali metal salts such as sodium salt and potassium salt, alkaline earth metal salts such as calcium salt and magnesium salt, salts with organic bases such as trimethylamine, triethylamine, pyridine, picoline, dicyclohexylamine, N,N'-dibenzylethylenediamine, arginine and lysine (organic amine salts), and ammonium salts.
Furthermore, the sulfonamide compound may be in anhydride form, and may form a solvate such as a hydrate. The solvate may be either a hydrate or a nonhydrate, preferably a hydrate. The solvent used may be water, alcohol (e.g., methanol, ethanol or n-propanol), dimethylformamide or the like.
If solvates and/or enantiomers of these compounds exist, the sulfonamide compound of the invention comprises these solvates and/or enantiomers. The sulfonamide compound of the invention also comprises a sulfonamide compound that undergoes metabolism such as oxidation, reduction, hydrolysis and conjugation *in vivo.* Moreover, the sulfonamide compound of the invention also comprises compounds that generate a sulfonamide compound by undergoing metabolism such as oxidation, reduction and hydrolysis *in vivo.*
According to the present invention, a sulfonamide compound is any one compound selected from the group consisting of those represented by Formulae (I) to (V), preferably at least one compound selected from the group consisting of E7070, E7820, LY186641, LY295501, LY-ASAP, LY573636 and CQS, more preferably at least one compound selected from the group consisting of E7070, E7820, LY295501 and LY573636, particularly preferably E7820.

2. Method for examining the sensitivity

The present invention provides a method for examining the sensitivity of a tumor cell to a sulfonamide compound.
A first embodiment of the invention is a method for examining the sensitivity of a tumor cell to a sulfonamide compound, the method comprising the steps of:

(a) determining the EGFR1 expression level in a tumor cell removed from a cancer patient before and after the administration of the sulfonamide compound; and
(b) judging that the tumor cell is highly sensitive to the sulfonamide compound if the EGFR1 expression level in the tumor cell removed after the administration of the sulfonamide compound is decreased compared to the EGFR1 expression level in the tumor cell removed before the administration of said compound.

According to the present invention, the sulfonamide compound may be used directly or used as a pharmaceutical composition. Such a pharmaceutical composition is not particularly limited as long as it comprises the sulfonamide compound.
Examples of such pharmaceutical compositions include oral drugs such as tablets, powder, granule, subtle granule, capsules, syrup agents, lozenges and inhalant drugs, external drugs such as suppository, ointment, eye ointment, strip agents, eye drops, nasal drops, ear drops skin patch and lotion, and injection agents.
The tumor cells are removed from a cancer patient before and after the sulfonamide compound is administered in an amount that allows determination of the sensitivity of the tumor cells.
The given dose of the sulfonamide compound differs depending on the degree of the symptom, age, sex, weight and sensitivity difference of the patient, administration mode, administration period, administration interval, and nature, prescription and type of the pharmaceutical formation, and the type of the active ingredient. For example, but without limitation, the dose of the sulfonamide compound is 10-6000 mg/day, preferably 30-4000 mg/day, more preferably 50-2000 mg/day for an adult (weight 60 Kg), which may be administered once to three times a day.
Tumor cells can be obtained from a cancer patient, for example, by surgical manipulation (e.g., biopsy) or blood draw.
Such tumor cells also comprise tumor cells in blood.
The amount of tumor cells collected from a cancer patient should be an amount that allows determination of the EGFR1 expression level.
Examples of the types of tumors include brain tumor, cervical cancer, esophageal cancer, tongue cancer, lung cancer, breast cancer, pancreas cancer, gastric cancer, small intestinal and duodenal cancer, large bowel cancer (colon cancer and rectal cancer), bladder cancer, renal cancer, liver cancer, prostate cancer, uterine cancer, ovarian cancer, thyroid grand cancer, gallbladder cancer, pharyngeal cancer, sarcoma (e.g., osteosarcoma, chondrosarcoma, Kaposi's sarcoma, myosarcoma, angiosarcoma, fibrosarcoma, etc.), leukemia (e.g., chronic myelocytic leukemia (CML), acute myelocytic leukemia (AML), chronic lymphocytic leukemia (CLL), acute lymphocytic leukemia (ALL), lymphoma, malignant lymphoma, multiple myeloma (MM), etc.) and melanoma, preferably large bowel cancer, non-small cell lung cancer and pancreas cancer.

Herein, "EGFR1" refers to Epidermal Growth Factor Receptor 1 (also referred to as "erbB1" or "HER1"), which is well known by those skilled in the art as one of 170 kDa single transmembrane tyrosine kinases (Current Opinion in Cell Biology., 11, 184-189, 1999).

For example, EGFR1 may be a polypeptide containing the amino acid sequence represented by SEQ ID NO: 1 (GenBank Accession No:NM_005228). Many mutant EGFR1 have been reported (Nature Genetics., 37, 1315-1316, 2005, Human Pathology, 36, 1127-1134, 2005). Hence, EGFR1 according to the present invention also comprises polypeptides comprising an amino acid sequence having one or more (e.g., one or several) amino acids deleted, substituted, added or varied by a combination thereof in the amino acid sequence represented by SEQ ID NO:1.

Examples of polypeptides comprising an amino acid sequence having one or more (e.g., one or several) amino acids deleted, substituted, added or varied by a combination thereof in the amino acid sequence represented by SEQ ID NO:1 include:

(i) an amino acid sequence having 1-9 (e.g., 1-5, preferably 1-3, more preferably 1-2 and still more preferably one) amino acids deleted from the amino acid sequence represented by SEQ ID NO:1;
(ii) an amino acid sequence having 1-9 (e.g., 1-5, preferably 1-3, more preferably 1-2 and still more preferably one) amino acids added to the amino acid sequence represented by SEQ ID NO:1;
(iii) an amino acid sequence having 1-9 (e.g., 1-5, preferably 1-3, more preferably 1-2 and still more preferably one) amino acids substituted with other amino acids in the amino acid sequence represented by SEQ ID NO:1; and
(iv) an amino acid sequence varied by a combination of (i) to (iii) above.

As used herein, "deletion" of an amino acid refers to a mutation where one or more amino acid residues in the sequence are deleted, including deletion of amino acid residues from the end of the amino acid sequence and deletion in the middle of the amino acid sequence.

As used herein, "addition" of an amino acid refers to a mutation where one or more amino acid residues are added to the sequence, including addition of amino acid residues to the end of the amino acid sequence and addition in the middle of the amino acid sequence. Addition in the middle of the sequence may also be referred to as "insertion".

As used herein, "substitution" of an amino acid refers to a mutation where one or more amino acid residues in the sequence are substituted with different types of amino acid residues.

The expression level of EGFR1 can be determined by a known method such as immunochemical techniques (e.g., immunohistochemical techniques, western blotting, flow cytometry, ELISA, EIA, RIA, etc.) and mass spectrometry, preferably an immunochemical technique, more preferably an immunohistochemical technique, western blotting, flow cytometry or ELISA, particularly preferably immunohistochemical technique, flow cytometry or western blotting. These techniques can be carried out according to a routine procedure.

The expression level of EGFR1 can be calculated, for example, as an EGFR1 amount in a unit volume of the tumor, an EGFR1 amount in a unit weight of the tumor or an EGFR1 amount in the tumor cell.

The EGFR1 expression level can also be calculated using as a measure the expression level of a protein constantly expressed in a cell. Examples of proteins constantly expressed in a cell include beta-actin and GAPDH. An expression level of a protein constantly expressed in a cell can be calculated in the same manner as a method for determining the EGFR1 expression level.

In this case, the EGFR1 expression level is calculated as an EGFR1 amount to an amount of proteins constantly expressed in the cell.

Hereinafter, an exemplary method for determining the EGFR1 expression level in a tumor cell will be described.

The EGFR1 expression level in a tumor cell can be determined by western blotting.

Western blotting can be carried out following a routine procedure (Cell Technology, Supp., Visual Experimental Note Series, Biotechnology Experiments Illustrated Vol. 5, Fear No Protein, Chap. I: SDS-PAGE, pp.13-62, Chap. IV: Western blotting, pp. 105-126, Shujunsha, 1997).

First, a cell lysate is prepared from tumor cells removed from a cancer patient. A tumor cell lysate can be prepared by a routine procedure. In order to prepare a tumor cell lysate, for example, various protease inhibitors (Leupeptin, p-APMSF, EDTA, o-NaV04) and 10% glycerol-containing cell lysis solution may be added to the tumor cell.

Then, the tumor cell lysate may be subjected to western blotting.

For example, an SDS sample buffer is firstly added to the tumor cell lysate. This sample is then subjected to electrophoresis following a routine procedure. Examples of electrophoresis include sodium dodecyl sulfate polyacrylamide gel electrophoresis (SDS-PAGE), non-reduced SDS-PAGE, native-PAGE, isoelectric focusing electrophoresis and two-dimensional electrophoresis, preferably SDS-PAGE. Membrane transfer may take place after the electrophoresis according to a routine procedure. Examples of membranes include a nitrocellulose membrane, a nylon membrane and a PVDF membrane, preferably a PVDF membrane.

Then, the membrane can be pretreated with a solution containing, for example, BSA, Triton-X100, tween20, skim milk or casein. The pretreating procedure is not particularly limited and it can appropriately be selected depending on the antibody used.

Then, the pretreated membrane is brought into contact with an antibody having affinity for EGFR1 (hereinafter, also referred to as an "anti-EGFR1 antibody"). The anti-EGFR1 antibody may be a commercially available antibody (e.g., from BD Biosciences, Santa Cruz Biotechnology, Cell Signaling, etc.) or it may be prepared. The anti-EGFR1 antibody may or may not be labeled with a labeling substance. If the anti-EGFR1 antibody is unlabeled, an antibody that recognizes said anti-EGFR1 antibody (hereinafter, also referred to as a "secondary antibody") may be brought into contact. Preferably, the secondary antibody is labeled with a labeling substance. Examples of such a labeling substance include enzymes such as alkaline phosphatase, peroxidase, glucose oxidase and beta-galactosidase, fluorescent substances such as FITC (fluorescein isothiocianate), Alexa488, PE, Rhodamine, Texas Red, Cy3, Cy5, Allophycocyanin, PharRed, DsRed, AmCyan, ZsGreen, ZsYellow, AsRed and HcRed, as well as biotin. When the labeling substance is biotin, avidin, streptavidin or the like can additionally be brought into contact. Preferably, said avidin, streptavidin or the like is labeled with a labeling substance. Examples of such a labeling substance include enzymes such as alkaline phosphatase, peroxidase, glucose oxidase and beta-galactosidase, and fluorescent substances such as FITC, Alexa488, PE, Rhodamine, Texas Red, Cy3, Cy5, Allophycocyanin, PharRed, DsRed, AmCyan, ZsGreen, ZsYellow, AsRed and HcRed. Conditions for reaction (e.g., the reaction solution, the antibody concentration, the reaction time, the reaction temperature, the washing operation, etc.) can appropriately be selected depending on the antibody used.

When the labeling substance is an enzyme, a substrate and/or a color-producing reagent is brought into contact with the membrane for color development, which is observed for determination of the EGFR1 expression level.

When the enzyme is peroxidase, for example, $H_2O_2$ or the like as the substrate and diaminobenzidine (DAB) or the like as the color-producing reagent may be brought into contact with a tissue section.

When the enzyme is alkaline phosphatase, for example, 5-bromo-4-chloro-3-indolyl phosphate or the like as the substrate and nitrobluetetrazorium or the like as the color-producing reagent may be brought into contact with a tissue section. When the enzyme is alkaline phosphatase, for example, CSPD (disodium 3-(4-methoxyspiro[1,2-dioxetane-3,2'-(5'-chloro)tricyclo[3.3.1.1$^{3,7}$]decan]-4-yl)phenyl phosphate) or the like as the color-producing reagent may be brought into contact with a tissue section for chemiluminescent reaction.

When the labeling substance is a fluorescent substance, excitation light is radiated onto a tissue section for luminescence so that this fluorescence may be observed for determination of the EGFR1 expression level.

Hence, the EGFR1 expression level in a tumor cell may be determined in this manner.

Hereinafter, another exemplary method for determining the EGFR1 expression level in a tumor cell will be described.

The EGFR1 expression level in a tumor cell may be determined by an immunohistochemical technique using an anti-EGFR1 antibody.

An immunohistochemical technique can be carried out following a routine procedure (Cell Technology, Supp., Visual Experimental Note Series, Biotechnology Experiments Illustrated Vol. 5, Fear No Protein, Chap. V: Immunostaining, pp. 127-163, Shujunsha, 1997).

First, a tissue section is prepared from tumor removed from a cancer patient. Such a tissue section comprises, for example, a cryosection and a paraffin section.

For example, the tumor removed from a cancer patient may be untreated or fixed. This tumor may also be embedded, for example, in OCT compound or the like.

Fixing can be carried out with formaldehyde, preferably 4% PFA/PBS(-), followed by replacement with 20% sucrose/phosphate buffer or the like.

These conditions can appropriately be selected according to the antibody used.

The tissue section can be mounted on a glass slide and pretreated to prepare for staining.

The pretreating procedure is not particularly limited and may appropriately be selected according to the antibody used. The tissue section may be pretreated with a solution containing, for example, xylene, formaldehyde, acetone, methanol or the like. The tissue section may also be pretreated with a solution containing, for example, BSA, Triton-X100, tween20, skim milk, casein or the like.

Then the pretreated tissue section is brought into contact with an anti-EGFR1 antibody. The anti-EGFR1 antibody may be a commercially available antibody or it may be prepared. The anti-EGFR1 antibody may or may not be labeled with a labeling substance. If the anti-EGFR1 antibody is unlabeled, an antibody that recognizes said anti-EGFR1 antibody (hereinafter, also referred to as a "secondary antibody") may be brought into contact. Preferably, the secondary antibody is labeled with a labeling substance. Examples of such a labeling substance include enzymes such as alkaline phosphatase, peroxidase, glucose oxidase and beta-galactosidase, fluorescent substances such as FITC (fluorescein isothiocianate), Alexa488, PE, Rhodamine, Texas Red, Cy3, Cy5, Allophycocyanin, PharRed, DsRed, AmCyan, ZsGreen, ZsYellow, AsRed and HcRed, as well as biotin. When the labeling substance is biotin, avidin, streptavidin or the like can additionally be brought into contact. Said avidin, streptavidin or the like is preferably labeled with a labeling

substance. Examples of such a labeling substance include enzymes such as alkaline phosphatase, peroxidase, glucose oxidase and beta-galactosidase, and fluorescent substances such as FITC, Alexa488, PE, Rhodamine, Texas Red, Cy3, Cy5, Allophycocyanin, PharRed, DsRed, AmCyan, ZsGreen, ZsYellow, AsRed and HcRed. Conditions for reaction (e.g., the reaction solution, the antibody concentration, the reaction time, the reaction temperature, the washing operation, etc.) can appropriately be selected depending on the antibody used.

When the labeling substance is an enzyme, a substrate and/or a color-producing reagent is brought into contact with a tissue section for color development, which is observed for determination of the EGFR1 expression level in a tumor cell.

When the enzyme is peroxidase, for example, $H_2O_2$ or the like as the substrate and diaminobenzidine (DAB) or the like as the color-producing reagent may be brought into contact with a tissue section.

When the enzyme is alkaline phosphatase, for example, 5-bromo-4-chloro-3-indolyl phosphate or the like as the substrate and nitrobluetetrazorium or the like as the color-producing reagent may be brought into contact with a tissue section. When the enzyme is alkaline phosphatase, for example, CSPD (disodium 3-(4-methoxyspiro[1,2-dioxetane-3,2'-(5'-chloro)tricyclo[3.3.1.1$^{3,7}$]decan]-4-yl)phenyl phosphate) or the like as the color-producing reagent may be brought into contact with a tissue section for chemiluminescent reaction.

When the labeling substance is a fluorescent substance, excitation light is radiated onto a tissue section for luminescence so that this fluorescence may be observed for determination of the EGFR1 expression level.

Hence, the EGFR1 expression level in a tumor cell may be determined in this manner.

The pretreated tissue section may also be subjected to nuclear staining with hematoxylin or methyl green.

Furthermore, the treated tissue section may be mounted with an aqueous mounting medium.

Hereinafter, another exemplary method for determining the EGFR1 expression level in a tumor cell will be described.

The EGFR1 expression level in a tumor cell can be determined by flow cytometry using an anti-EGFR1 antibody.

Flow cytometry can be carried out following a routine procedure.

A tumor cell can be separated from a cancer tissue removed from a cancer patient by biopsy or the like using a routine procedure. For example, according to Hamburger et al. (Cancer Research., 38, 3438-3443, 1978), the obtained tissue is cut under sterile conditions so as to prepare a cell suspension using stainless mesh, syringe needle, nylon mesh or the like.

Then, the resulting tumor cell is brought into contact with an anti-EGFR1 antibody. The anti-EGFR1 antibody may be a commercially available antibody or it may be prepared. The anti-EGFR1 antibody may or may not be labeled with a labeling substance. If the anti-EGFR1 antibody is unlabeled, an antibody that recognizes said anti-EGFR1 antibody (hereinafter, also referred to as a "secondary antibody") may be brought into contact. Preferably, the secondary antibody is labeled with a labeling substance. Examples of such a labeling substance include fluorescent substances such as FITC (fluorescein isothiocianate), Alexa488, PE, Rhodamine, Texas Red, Cy3, Cy5, Allophycocyanin, PharRed, DsRed, AmCyan, ZsGreen, ZsYellow, AsRed and HcRed, as well as biotin. When the labeling substance is biotin, avidin, streptavidin or the like can be brought into contact. Said avidin, streptavidin or the like is preferably labeled with a labeling substance. Examples of such a labeling substance include fluorescent substances such as FITC, Alexa488, PE, Rhodamine, Texas Red, Cy3, Cy5, Allophycocyanin, PharRed, DsRed, AmCyan, ZsGreen, ZsYellow, AsRed and HcRed. The cell may be either untreated or fixed. Fixing can be carried out, for example, with methanol, acetone or formaldehyde, preferably 4% PFA/PBS(-). Conditions for reaction (e.g., the reaction solution, the antibody concentration, the reaction time, the reaction temperature, the washing operation, the fixing treatment, etc.) can appropriately be selected depending on the antibody used.

Determination of the fluorescence intensity in the tumor cell by flow cytometry results in determination of the EGFR1 expression level in the tumor cell.

Hereinafter, another exemplary method for determining the EGFR1 expression level in a tumor cell will be described.

The EGFR1 expression level in a tumor cell can be determined by ELISA using an anti-EGFR1 antibody.

ELISA can be carried out following a routine procedure. Alternatively, a commercially available determination kit may be used (e.g., DAKO Cytomation).

First, a cell lysate is prepared with tumor cells removed from a cancer patient. A tumor cell lysate can be prepared by a routine procedure. In order to prepare a tumor cell lysate, various protease inhibitors (Leupeptin, p-APMSF, EDTA, o-NaV04) and a 10% glycerol-containing cell lysis solution may be added to the tumor cell.

Then, the tumor cell lysate may be subjected to ELISA.

First, the-cell lysate is placed into a plate (e.g., 96-well plate, 384-well plate, etc.). The plate is not particularly limited but preferably a plate having an-anti-EGFR1 antibody solid-phased thereon. Preferably, standard EGFR1 is placed in a different well from the cell lysate. Then, an anti-EGFR1 antibody is added to each well. The anti-EGFR1 antibody may be a commercially available antibody or it may be prepared. The anti-EGFR1 antibody may or may not be labeled with a labeling substance. If the anti-EGFR1 antibody is unlabeled, an antibody that recognizes said anti-EGFR1 antibody (hereinafter, also referred to as a "secondary antibody") may be brought into contact. Preferably, the secondary antibody is labeled with a labeling substance. Examples of such a labeling substance include enzymes such as alkaline phosphatase, peroxidase, glucose oxidase and beta-galactosidase, fluorescent substances such as FITC (fluorescein isothiocianate), Alexa488, PE, Rhodamine, Texas Red, Cy3, Cy5, Allophycocyanin, PharRed, DsRed, AmCyan, ZsGreen, ZsYellow, AsRed and HcRed, as well as biotin. When the labeling substance is biotin, avidin, streptavidin or the like can be brought into contact. Said avidin, streptavidin or the like is preferably labeled with a labeling substance. Examples of such a labeling substance include enzymes such as alkaline phosphatase, peroxidase, glucose oxidase and beta-galactosidase, and fluorescent substances such as FITC, Alexa488, PE, Rhodamine, Texas Red, Cy3, Cy5, Allophycocyanin, PharRed, DsRed, AmCyan, ZsGreen, ZsYellow, AsRed and HcRed. Conditions for reaction (e.g., the reaction solution, the antibody concentration, the reaction time, the reaction temperature, the washing operation, etc.) can appropriately be selected depending on the antibody used.

When the labeling substance is an enzyme, a substrate and/or a color-producing reagent is brought into contact with the membrane for color development, which is observed for determination of the EGFR1 expression level.

When the enzyme is peroxidase, for example, $H_2O_2$ or the like as the substrate and diaminobenzidine (DAB) or the like as the color-producing reagent may be brought into contact with a tissue section.

When the enzyme is alkaline phosphatase, for example, 5-bromo-4-chloro-3-indolyl phosphate or the like as the substrate and nitrobluetetrazorium or the like as the color-producing reagent may be brought into contact with a tissue section. When the enzyme is alkaline phosphatase, for example, CSPD (disodium 3-(4-methoxyspiro[1,2-dioxetane-3,2'-(5'-chloro)tricyclo[3.3.1.1$^{3,7}$]decan]-4-yl)phenyl phosphate) or the like as the color-producing reagent may be brought into contact with a tissue section for cheniiluminescent reaction.

When the labeling substance is a fluorescent substance, excitation light is radiated onto a tissue section for luminescence so that this fluorescence may be observed for determination of the EGFR1 expression level.

Hence, the EGFR1 expression level in a tumor cell may be determined in this manner.

Although use of an anti-EGFR1 antibody has been described herein for determining the EGFR1 expression level, an antibody having affinity for phosphorylated EGFR1 (hereinafter, also referred to as an "anti-phosphorylated-EGFR1 antibody") may be used instead of an anti-EGFR1 antibody in the present invention to quantify the phosphorylation level of EGFR1 for determination of the EGFR1 expression level. The anti-phosphorylated-EGFR1 antibody may be a commercially available antibody (e.g., Cell Signaling) or it may be prepared.

The EGFR1 expression level in a tumor cell may also be determined by mass spectrometry (e.g., by the method described in WO2005/050188). In this case, an anti-EGFR1 antibody or an anti-phosphorylated-EGFR1 antibody is used for operation according to a routine technique to determine the EGFR1 expression level.

A second aspect of the invention is a method for examining the sensitivity of a tumor cell to a sulfonamide compound, comprising the steps of:

(a) treating the tumor cell removed from a cancer patient with the sulfonamide compound;
(b) determining the EGFR1 expression level in the tumor cell treated in step (a) and an untreated tumor cell; and
(c) judging that the tumor cell is highly sensitive to the sulfonamide compound if the EGFR1 expression level in the tumor cell treated in step (a) is decreased compared to the EGFR1 expression level in the untreated tumor cell.

In this aspect, the tumor cells removed from a cancer patient that are used for examining the sensitivity thereof to a sulfonamide compound may be a tumor cell cultured after collection and treated with the sulfonamide compound, and an untreated tumor cell. For treating the tumor cell with a sulfonamide compound, for example 0.01-100 $\mu$M, preferably 0.1-10$\mu$M, more preferably 0.3-3$\mu$M of said compound is brought into contact with 1 x $10^6$ cells. The untreated cell is a cell that has not been treated with the compound. Conditions for treating the tumor cell with a sulfonamide compound include a treating time of, for example, 3-72 hours, preferably 6-48 hours, more preferably 12-24 hours and a temperature of, for example, 25-38°C, preferably 37°C.

In this aspect, the tumor cell removed from a cancer patient may be separated following a routine procedure from a cancer tissue removed from the cancer patienfby biopsy. For example, according to Hamburger et al. (Cancer Research., 38, 3438-3443, 1978), the obtained tissue is cut under sterile conditions so as to prepare a cell suspension using stainless mesh, syringe needle, nylon mesh or the like. The resulting cell may be cultured in an appropriate medium (e.g., RPMI-1640, MEM or McCoy medium containing 10-15% FCS). Alternatively, a tumor cell may exclusively be separated specifically from the tissue used obtained by biopsy or the like by soft agar culture (Science, 197, 461-463, 1977, Journal of Clinical Investigation., 60, 846-854, 1977, Proceeding, American. Association. Cancer Research., 20, 51, 1979).

A method for determining the EGFR1 expression level can be carried out according to a known method such as immunochemical techniques (e.g., immunohistochemical techniques, western blotting, flow cytometry, ELISA, EIA, RIA, etc.) and mass spectrometry, preferably an immunochemical technique, more preferably flow cytometry or ELISA, particularly preferably flow cytometry. These techniques can be carried out according to a routine procedure.

An expression level of EGFR1 can be calculated, for example, as an EGFR1 amount in a unit volume of the tumor, an EGFR1 amount in a unit weight of the tumor or an EGFR1 amount in the tumor cell.

According to the present invention, if the EGFR1 expression level is decreased, the tumor cell is judged to be highly sensitive to the sulfonamide compound.

Specifically, according to the present invention, a cancer patient is judged to be highly sensitive to a sulfonamide, if the EGFR1 expression level in a tumor cell removed from a cancer patient after the administration of the sulfonamide compound is decreased compared to the EGFR1 expression level in a tumor cell removed from the patient before the administration of the sulfonamide compound and/or when the EGFR1 expression level in a tumor cell removed from a cancer patient and treated with the sulfonamide compound is decreased compared to the EGFR1 expression level in an untreated tumor cell.

The EGFR1 expression level is judged to have "decreased" when the EGFR1 expression level is decreased by 1.2, preferably 1.5, more preferably 1.8 and particularly preferably 2.0 times or more. The difference in decrease may also be 2.5, 3.0, 3.5 or 4.0 times or more.

According to the present invention, when a tumor cell is judged to be highly sensitive to a sulfonamide compound; the sulfonamide compound is judged to have a higher anti-tumor effect on the cancer patient having said tumor cell. Exemplary cases of higher anti-tumor effects refer to, for example, an anti-tumor effect that is expected to be higher than an average anti-tumor effect for patients with similar symptoms, an anti-tumor effect that is expected to be higher than that for other patients with the same type of cancer, or an anti-tumor effect that is expected to be higher than that for patients with other types of cancer. Hence, judgment of the presence or the absence of a higher anti-tumor effect is highly useful in clinical practice since cancer patients can be selected who could be prospective candidates for obtaining higher anti-tumor effects.

A third aspect of the invention is a method for examining the sensitivity of a tumor cell to a sulfonamide compound, comprising the steps of:

(a) determining the EGFR1 expression levels in the tumor cells of a cancer patient before and after the administration of the sulfonamide compound or the analog thereof; and
(b) judging that the tumor cell is highly sensitive to the sulfonamide compound or the analog thereof if the EGFR1 expression level in the tumor cell after the administration of the sulfonamide compound or the analog thereof is decreased compared to the EGFR1 expression level in the tumor cell before the administration of said compound.

In this aspect, sensitivity to a sulfonamide compound can be determined without removing tissue cells from the cancer patient. Specifically, in the step of determining the EGFR1 expression levels, an anti-EGFR1 antibody labeled with a positron-emitting radionuclide or a radioactive substance is administered before and after the administration of the sulfonamide compound to the patient, and the expression levels of the labeled anti-EGFR1 antibody were detected using a nuclear medicine scanning device such as PET (Positron Emission Tomography) device or SPECT (Single Photon Emission Computed Tomography) device to determine the EGFR1 expression level in the body of the patient before and after the administration of the sulfonamide compound.

A fourth aspect of the invention is a method for examining the sensitivity of a tumor cell to a sulfonamide compound, comprising the steps of:

(a) determining the expression levels of EGFR1 in soluble form in blood drawn from a cancer patient before and after the administration of the sulfonamide compound or the analog thereof; and
(b) judging that the tumor cell is highly sensitive to the sulfonamide compound or the analog thereof if the expression level of EGFR1 in soluble form in blood drawn after the administration of the sulfonamide compound or the analog thereof is decreased compared to the expression level of EGFR1 in soluble form in blood drawn before the administration of the compound.

Herein, "EGFR1 in soluble form" refers to isoforms of EGFR1 present in blood and the protein of the domain outside the membrane forming EGFR1.

Based on the fact that EGFR1 in soluble form is present in blood as reported by A.T.Baron et al. J. Immunol. Methods, 219:23-43, 1998, the present aspect allows examination of the sensitivity of a tumor cell to a sulfonamide compound by calculating the expression level of the EGFR1 in soluble form present in blood in a way similar to that for the EGFR1 expression level in a tumor cell.

The expression level of the EGFR1 in soluble form present in blood can be calculated by determining the expression level of the EGFR1 in soluble form in the blood drawn from a caner patient in the same manner as the first aspect of the invention by the method for determining the EGFR1 expression level, preferably ELISA.

The EGFR1 in soluble form in blood can be calculated, for example, as an amount of EGFR1 in soluble form

in a unit volume of blood or an amount of EGFR1 in soluble form in a unit weight of blood.

In order to determine the expression level of EGFR1 in soluble form present in blood by ELISA, serum is separated from blood and suitably diluted where appropriate. A commercially available determination kit may be used for this dilution (e.g., DAKO Cytomation). In addition, according to a routine procedure, an expression level of EGFR1 in soluble form in blood can be determined by observing color development by enzyme reaction or fluorescence excited by excitation light in the same manner as in the first aspect of the invention.

In the first, third and fourth aspects of the invention, when a tumor cell is judged to be highly sensitive to a sulfonamide compound, the sulfonamide compound may be judged to have an anti-tumor effect on a cancer patient having that tumor cell. Hence, the present invention also provides a method for examining an anti-tumor effect of a sulfonamide compound.

Thus, a fifth aspect of the invention is a method for examining an anti-tumor effect of a sulfonamide compound, comprising the steps of:

(a-1) determining the EGFR1 expression levels in tumor cells removed from a cancer patient before and after the administration of the sulfonamide compound; and
(b-1) judging that the sulfonamide compound is exerting an anti-tumor effect if the EGFR1 expression level in the tumor cell removed after the administration of the sulfonamide compound is decease compared to the EGFR1 expression level in the tumor cell removed before the administration of said compound,
OR
(a-2) determining the EGFR1 expression levels in tumor cells of a cancer patient before and after the administration of the sulfonamide compound or the analog thereof; and
(b-2) judging that the sulfonamide compound or the analog thereof is exerting an anti-tumor effect if the EGFR1 expression level in the tumor cell after the administration of the sulfonamide compound or the analog thereof is decreased compared to the EGFR1 expression level in the tumor cell before the administration of said compound,
OR
(a-3) determining the expression levels of EGFR1 in soluble form in blood drawn from a cancer patient before and after the administration of the sulfonamide compound or the analog thereof; and
(b-3) judging that the sulfonamide compound or the analog thereof is exerting an anti-tumor effect if the expression level of EGFR1 in soluble form in blood drawn after the administration of the sulfonamide compound or the analog thereof is decreased compared to the expression level of EGFR1 in soluble form in blood drawn before the administration of said compound.

Accordingly, judgment of the presence or the absence of an anti-tumor effect in a short time is highly useful in determining an appropriate dosage and the like in clinical practice.

3. Test kit

The present invention provides a test kit used in a method for examining the sensitivity of a tumor cell to a sulfonamide compound and/or a method for examining an anti-tumor effect of a sulfonamide compound, the kit comprising an antibody having affinity for EGFR1 (also referred to as an "anti-EGFR1 antibody") and/or an antibody having affinity for phosphorylated EGFR1 (also referred to as an "anti-phosphorylated-EGFR1 antibody"). The antibody can be prepared by a conventional technique. A commercially available anti-EGFR1 antibody (e.g., BD Biosciences, Santa Cruz Biotechnology, Cell Signaling) and/or anti-phosphorylated-EGFR1 antibody (e.g., Cell Signaling) may be purchased. The test kit of the invention may comprise, in addition to the antibodies mentioned above, components conventionally used in general determination. In addition to the antibody mentioned above, the test kit of the invention may also comprise an instruction, a packaging insert or the like describing use of the anti-EGFR1 antibody and/or the anti-phosphorylated-EGFR1 antibody in a method for examining the sensitivity of a tumor cell to a sulfonamide compound and/or a method for examining an anti-tumor effect of a sulfonamide compound.

Hereinafter, the present invention will be described by way of specific examples, although the present invention should not be limited thereto.

[EXAMPLE 1]

<u>Anti-tumor effect of E7820 on subcutaneous transplant models (*in vivo*) of human cancer cell lines</u>

Human non-small-cell lung cancer cell line PC9 (obtained from Immuno-Biological laboratories Co., Ltd.), human non-small-cell lung cancer cell line A549 (obtained from Dainippon Pharmaceutical), human colon cancer cell line WiDr (obtained from Dainippon Pharmaceutical) and human colon cancer cell line HCT116 (obtained from ATCC)

were cultured in RPMI1640 (containing 10% FBS) in a 5% carbon dioxide incubator at 37°C to about 80% confluence, and the cells were collected with trypsin-EDTA. Using a phosphate buffer for PC9, WiDr and HCT116 and a 50% matrigel-containing phosphate buffer for A549, 5 x $10^7$ cells/mL suspensions were prepared, respectively. Then, 0.1 mL each of the resulting cell suspensions was subcutaneously transplanted to the side of a nude mouse.

For PC9, 8 days after the transplantation, E7820 was orally administered for 50 mg/kg, 100 mg/kg or 200 mg/kg twice a day for 14 days. For A549, 24 days after the transplantation, E7820 was orally administered for 50 mg/kg or 200 mg/kg twice a day for 7 days. For WiDr, 11 days after the transplantation, E7820 was orally administered for 50 mg/kg, 100 mg/kg or 200 mg/kg twice a day for 14 days. For HCT116, 23 days after the transplantation, E7820 was orally administered for 6.25 mg/kg, 12.5 mg/kg, 25 mg/kg, 50 mg/kg, 100 mg/kg or 200 mg/kg twice a day for 14 days.

The major and minor axes of tumors were measured with Digimatic caliper (Mitsutoyo), and tumor volumes and relative tumor volumes were calculated according to the following formulae.

$$\text{Tumor Volume (TV)} = \text{Major axis of tumor (mm)} \times (\text{Minor axis of tumor})^2 (\text{mm}^2)/2$$

$$\text{Relative Tumor Volume (RTV)} = \text{Tumor volume on measurement day/Tumor volume on the first administration day}$$

As a result, although E7820 showed anti-tumor effects for all human cancer cell lines, their levels varied (Figures 1-4). Specifically, E7820 showed stronger anti-tumor effects for PC9 and HCT166 than for A549 and WiDr.

[EXAMPLE 2]

<u>Effect of E7820 on EGFR1 expression level in cultured human cancer cell lines (*in vitro*) (flow cytometry)</u>

PC9 or A549 was suspended in RPMI1640 (containing 10% FBS) to 1 x $10^5$ cells/ml. Then, 1 ml each of these suspensions was placed into each well of a 6-well plate and cultured in a 5% carbon dioxide incubator at 37°C. On the following day, 1 ml each of E7820 culture dilutions at respective concentrations (0-20 μM) was added to the cells in each well for further culture. After two days of culture, the cells were collected with trypsin-EDTA according to a routine procedure, which were suspended in PBS containing 0.5% BSA. Subsequently, an anti-EGFR1 antibody (BD Biosciences) was added to these suspensions and incubated at 4°C for 30 minutes. Centrifugation and washing with PBS were carried out according to routine procedures to remove an excessive amount of the antibody. Then, an RPE-labeled anti-mouse IgG antibody was added to these suspensions and incubated at 4°C for 30 minutes. Centrifugation and washing with PBS were carried out according to routine procedures to remove an excessive amount of the antibody. Finally, the EGFR1 expression level in PC9 and A549 were determined with FACSCalibur (Japan Becton, Dickinson and Company).

As a result, E7820 greatly decreased the EGFR1 expression level for human non-small-cell lung cancer cell line PC9 in a dose-dependent manner (Table 1) whereas E7820 slightly decreased the EGFR1 expression level in human non-small-cell lung cancer cell line A549 (Table 1). That is to say, E7820 decreased the EGFR1 expression level to a much greater extent for the highly sensitive cancer cell line (PC9) than for the less sensitive cancer cell line (A549).

Table 1

| Concentration of E7820 (μM) | 0 | 0.01 | 0.03 | 0.1 | 0.3 | 1 | 3 | 10 |
|---|---|---|---|---|---|---|---|---|
| EGFR1 expression level (% of control) PC9 | 100 | 95 | 91 | 80 | 65 | 49 | 44 | 37 |
| EGFR1 expression level (% of control) A549 | 100 | 93 | 97 | 95 | 91 | 80 | 64 | 73 |

Table 1 shows an effect of E7820 on the EGFR1 expression level for cultured human non-small-cell lung cancer cell line (PC9 or A549).

As can be evidenced from the above results, we were able to examine the sensitivity of a tumor cell to E7820 by using the EGFR1 expression level as a measure.

[EXAMPLE 3]

Effect of E7820 on EGFR1 expression level in subcutaneous transplant models (*in vivo*) of human cancer cell lines (western blotting)

PC9 was cultured in RPMI1640 (containing 10% FBS) in a 5% carbon dioxide incubator to about 80% confluence. The cells were collected with trypsin-EDTA according to a routine procedure and 5 x $10^7$ cells/mL suspension was prepared with a phosphate buffer. Then, 0.1 mL each of the resulting suspension was subcutaneously transplanted to the side of a nude mouse. Eight days after the transplantation, E7820 was orally administered for 50 mg/kg or 200 mg/kg twice a day for a week. One week after the start of sadministration, tumors were removed to prepare tumor cell lysates with various protease inhibitors and a cell lysis solution containing 10% glycerol and 1% Triton X-100 in ice. For each tumor cell lysate, an equal amount of protein was fractionated by SDS-PAGE and transferred onto a nitrocellulose membrane (Hybond ECL, Amersham Biosciences). Then, western blotting was performed by using an anti-EGFR1 antibody (Santa Cruz Biotechnology) or an anti-EGFR pY1068 antibody (anti-phosphorylated-EGFR1 antibody) (Cell Signaling) and horse radish peroxidase-labeled anti-mouse IgG antibody (secondary antibody) (Cell Signaling) according to a routine procedure.

Detection and quantification of each band were carried out as follows. First, each band was forced to chemically develop a color with Chemiluminescent Substrate (PIERCE), which was then captured as a digital image with Image Master VDS-CL (Amersham Pharmacia Biotech, Tokyo). The digital images were saved as TIF files and each band was numerically converted with Image Master total Lab (version 1.00, Amersham Pharmacia Biotech, Tokyo).

As a result, for transplanted tumors, E7820 again decreased the EGFR1 expression level and inhibited phosphorylation of EGFR1 in human non-small cell lung cancer cell line PC9 in a dose-dependent manner (Table 2).

Table 2

| E7820 dosage (mg/kg) | 0 | 50 | 200 |
|---|---|---|---|
| EGFR1 expression level (% of control) | 100 | 63 | 40 |
| Phosphorylation level of EGFR1 (% of control) | 100 | 59 | 51 |

Table 2 shows the effect of E7820 on the expression level and the phosphorylation level of EGFR1 in the subcutaneous transplant models of human non-small cell lung cancer cell lines (PC9).

As can be evidenced from the above results, we were able to examine the sensitivity of a tumor cell to E7820 and/or the anti-tumor effect of E7820 by using the EGFR1 expression level and/or the EGFR1 phosphorylation level as a measure.

[EXAMPLE 4]

Effect of E7820 on EGFR1 expression level in subcutaneous transplant models (*in vivo*) of human cancer cell lines (western blotting)

HCT116 or WiDr was cultured in RPMI1640 (containing 10% FBS) in a 5% carbon dioxide incubator to about 80% confluence. The cells were collected with trypsin-EDTA according to a routine procedure and 5 x $10^7$ cells/mL suspensions were prepared with a phosphate buffer. Then, 0.1 mL each of the resulting suspension was subcutaneously transplanted to the side of a nude mouse.

For HCT116, 23 days after the transplantation, E7820 was orally administered for 12.5 mg/kg, 50 mg/kg or 200 mg/kg twice a day for 3 days. On the other hand, for WiDr, 18 days after the transplantation, E7820 was orally administered for 50 mg/kg or 200 mg/kg twice a day for 7 days. Four days after the start of administration for HCT116 and 8 days after the start of administration for WiDr, tumors were removed to prepare tumor cell lysates with various protease inhibitors and a cell lysis solution containing 10% glycerol and 1% Triton X-100 in ice. For each tumor cell lysate, an equal amount of protein was fractionated by SDS-PAGE and transferred onto a nitrocellulose membrane (Hybond ECL, Amersham Biosciences). Then, western blotting was performed by using an anti-EGFR1 antibody (Santa Cruz Biotechnology) and horse radish peroxidase-labeled anti-mouse IgG antibody (secondary antibody) (Cell Signaling) according to a routine procedure.

Detection and quantification of each band were carried out as follows. First, each band was forced to chemically develop a color with Chemiluminescent Substrate (PIERCE), which was then captured as a digital image with Image Master VDS-CL (Amersham Pharmacia Biotech, Tokyo). The digital images were saved as TIF files and numerically converted with Image Master total Lab (version 1.00, Amersham Pharmacia Biotech, Tokyo).

As a result, whereas E7820 decreased the EGFR1 expression level in human colon cancer cell line HCT116 in a dose-dependent manner (Table 3), E7820 did not change the EGFR1 expression level in human colon cancer cell line WiDr (Table 3). That is to say, E7820 did not change the EGFR1 expression level in the less sensitive cancer cell line (WiDr) but only decreased the EGFR1 expression level in the highly sensitive cancer cell line (HCT116).

Table 3

| E7820 dosage (mg/kg) | 0 | 12.5 | 50 | 200 |
|---|---|---|---|---|
| EGFR1 expression level (% of control) HCT116 | 100 | 37 | 47 | 42 |
| EGFR1 expression level (% of control) WiDr | 100 | - | 117 | 121 |

Table 3 shows the effect of E7820 on the EGFR1 expression level in the subcutaneous transplant models of human colon cancer cell lines (HCT116 or WiDr).

As can be evidenced from the above results, we were able to examine the sensitivity of a tumor cell to E7820 and/or the anti-tumor effect of E7820 by using the EGFR1 expression level as a measure.

[EXAMPLE 5]

Effect of E7820 on EGFR1 expression level in subcutaneous transplant models (*in vivo*) of human cancer cell lines (immunohistochemical staining)

PC9 was cultured in RPMI1640 (containing 10% FBS) in a 5% carbon dioxide incubator at 37°C. The cultured human tumor cells (PC9) (8 x $10^6$ cells/mouse) were subcutaneously transplanted into nude mice (KSN mice from SLC). Once proliferation of the tumors transplanted into the mice was observed, E7820 was orally administered to the mice for 0 mg/kg (unadministered), 25 mg/kg, 50 mg/kg, 100 mg/kg or 200 mg/kg twice a day for 7 days. Following 7 days of administration, tumor tissues were removed from the mice, fixed with formalin and embedded in paraffm. Subsequently, the paraffin-embedded tumor tissues were sliced into a thickness of 6 μm, placed on glass slides and deparaffinized with xylene/ethanol. Then, these sections were treated with protein kinase K (Roche), and subjected to immunostaining according to the protocol of Simple Stain kit: mouse MAX-PO (M) (NICHIREI) using an anti-EGFR antibody (DakoCytomation). The results are shown in Figure 5. Referring to Figure 5, (a) to (f) show stain with control IgG (negative control), E7820-unadministered group, E7820-administered group (25 mg/kg), E7820-administered group (50 mg/kg), E7820-administered group (100 mg/kg) and E7820-administered group (200 mg/kg), respectively. Each panel was enlarged twenty times. As a result, a decrease in the EGFR1 expression level was significant for the E7820-administered groups (Figures 5(c) to 5(f)) as compared to the E7820-unadministered group (vehicle) (Figure 5(b)). This proves that E7820 also exerts an anti-tumor effect at tissue level by decreasing the EGFR1 expression level. The present example strongly supports that sensitivity of a tumor cell to a sulfonamide compound and/or an anti-tumor effect of a sulfonamide compound can be examined by using the EGFR1 expression level as a measure.

[EXAMPLE 6]

Effect of E7070 on EGFR1 expression level in cultured human cancer cell lines (*in vitro)* (mass spectrometry)

First, RPMI-1640 (containing 10% FBS) supplemented with $^{13}$C-labeled L-leucine ($^{13}$C-leucine, Cambridge Isotope Labs) and RPMI-1640 (containing 10% FBS) supplemented with non-isotope-labeled L-leucine ($^{12}$C-leucine) were prepared.

Then, human colon cancer cell line HCT116-C9 was cultured in each medium through at least the fifth passage. HCT116-C9 was a subline separated from human colon cancer cell line HCT116 (obtained from ATCC) and known as a cell line highly sensitive to E7070 (Molecular Cancer Therapeutics, 2002, 1, 275-286). Thereafter, each cell was seeded in a 15-cm-diameter culture dish at a concentration of 1.0 x $10^7$ cells/50 ml.

Then, 24 hours later, 1 μM of E7070 was added to HCT116-C9 cultured in the $^{12}$C-leucine-containing medium and nothing was added to HCT116-C9 cultured in the $^{13}$C-leucine-containing medium. These cells were continued culturing.

Twenty-four hours later, the cells were washed twice with a phosphate buffer saline according to a routine procedure, added with 3 ml of M-PER (PIERCE) to solubilize the cells and then equal amounts of them were mixed. The cell lysates were dialyzed by a routine procedure to sublimate the solutions, and digested with trypsin, thereby obtaining peptide samples. The resulting peptide samples were analyzed with LC-MS/MS. Next, for those having a ratio of a peak intensity of the peptide fragment containing $^{12}$C-leucine to a peak intensity of the peptide fragment

containing $^{13}C$-leucine ($^{12}C/^{13}C$) of 1 or less, the proteins were exhaustively identified based on the sequence information of the peptide fragments. This means that peptides with a peak intensity ratio ($^{12}C/^{13}C$) of 1 or less were derived from proteins whose expression levels have been decreased with 1 μM of E7070 in HCT116-C9.

As a result, a peak intensity ratio of 0.6-0.8 was observed for the EGFR1-derived peptide fragment such as ELVEPLTPSGEAPNQALLR, meaning that E7070 also decreased the EGFR1 expression level in human colon cancer cell line HCT116-C9.

As can be evidenced from the above results, we were able to examine the sensitivity of a tumor cell to E7070 and/or the anti-tumor effect of E7070 by using EGFR1 expression level as a measure.

In addition, E7070, E7820, LY186641, LY295501, LY-ASAP, LY573636, CQS and a combination thereof have been reported to have identical or similar mechanisms and thus give identical or similar actions and effects (WO2006/030941, WO2006/030947 and WO2006/036025). From this fact and the previous results (Examples 1-6), we were able to examine the sensitivity of a tumor cell to a sulfonamide compound and/or the anti-tumor effect of a sulfonamide compound by using the EGFR1 expression level as a measure.

INDUSTRIAL APPLICABILITY

The present invention enables examination of the sensitivity of a tumor cell to a sulfonamide compound.

More specifically, the present invention enables examination of the sensitivity of a tumor cell to a sulfonamide compound, preferably E7070, E7820, LY186641, LY295501, LY-ASAP, LY573636, CQS or a combination thereof, by using change in the EGFR1 expression levels as a measure based on comparison between the EGFR1 expression level in a tumor cell removed from a cancer patient after the administration of the sulfonamide compound and the EGFR1 expression level in a tumor cell removed from the cancer patient before the administration of the sulfonamide compound and/or by using change in the EGFR1 expression level as a measure based on comparison between the EGFR1 expression level in a tumor cell removed from a cancer patient and treated with the sulfonamide compound and the EGFR1 expression level in an untreated tumor cell.

Examination of the sensitivity of a tumor cell to a sulfonamide compound is highly useful in clinical practice in that cancer patients can be selected who could be prospective candidates for obtaining higher anti-tumor effects.

In addition, the present invention enables examination of an anti-tumor effect of a sulfonamide compound.

Specifically, the present invention enables examination of an anti-tumor effect of a sulfonamide compound, preferably E7070, E7820, LY186641, LY295501, LY-ASAP, LY573636, CQS or a combination thereof, by using change in the EGFR1 expression levels as a measure based on comparison between the EGFR1 expression level in a tumor cell removed from a cancer patient after the administration of the sulfonamide compound and the EGFR1 expression level in a tumor cell removed from the cancer patient before the administration of the sulfonamide compound.

Examination of an anti-tumor effect of a sulfonamide compound is highly useful in determining an appropriate dosage or the like in clinical practice.

## Claims

1. A method for examining the sensitivity of a tumor cell to a sulfonamide compound or an analog thereof, comprising the steps of:

   (a) determining the EGFR1 expression level in a tumor cell removed from a cancer patient before and after the administration of the sulfonamide compound or the analog thereof; and
   (b) judging that the tumor cell is highly sensitive to the sulfonamide compound or the analog thereof if the EGFR1 expression level in the tumor cell removed after the administration of the sulfonamide compound or the analog thereof is decreased compared to the EGFR1 expression level in the tumor cell removed before the administration of said compound,

   wherein the sulfonamide compound or the analog thereof is at least one compound selected from the group consisting of:

   a compound represented by General Formula (I)

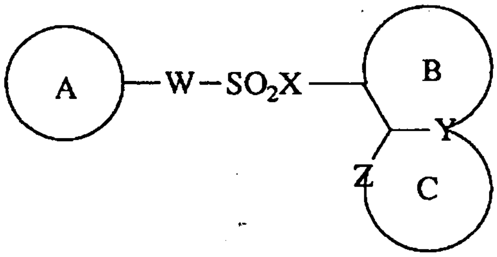

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents -N($R^1$)- or an oxygen atom,
Y represents a carbon atom or a nitrogen atom, and
Z represents -N($R^2$)- or a nitrogen atom,

wherein, $R^1$ and $R^2$ each independently represent, identically or differently, a hydrogen atom or a lower alkyl group];
a compound represented by General Formula (II)

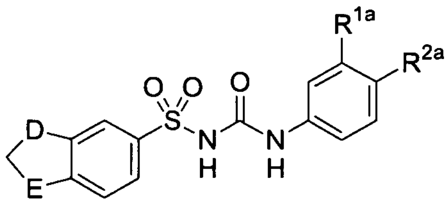

(II)

[wherein, E represents -O-, -N($CH_3$)-, -$CH_2$-, -$CH_2CH_2$- or -$CH_2O$-, D represents -$CH_2$- or -O-, $R^{1a}$ represents a hydrogen atom or a halogen atom, and $R^{2a}$ represents a halogen atom or a trifluoromethyl group];
a compound represented by General Formula (III)

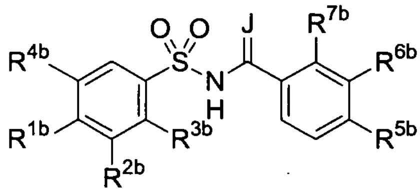

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, -$CF_3$, -$OCF_3$; -$SCF_3$, an optionally substituted $C_1$-$C_4$ alkoxy carbonyl group, a nitro group, an azido group, -O($SO_2$)$CH_3$, -N($CH_3$)$_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, -$CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, -$CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or -$CF_3$ (provided that when either one of $R^{5b}$ or $R^{7b}$ is an optionally substituted $C_1$-$C_6$ alkyl group or when $R^{7b}$ is a halogen atom or an optionally substituted

$C_1$-$C_6$ alkyl group, either one of $R^{5b}$ or $R^{6b}$ is a hydrogen atom)];
a compound represented by Formula (IV)

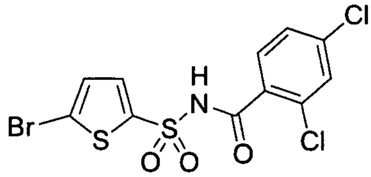

(IV)

;

and
a compound represented by Formula (V)

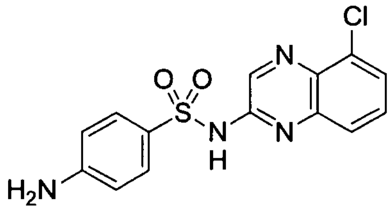

(V)

,

or a pharmacologically acceptable salt thereof, or a solvate thereof.

**2.** A method for examining the sensitivity of a tumor cell to a sulfonamide compound or an analog thereof, comprising the steps of:

(a) treating a tumor cell removed from a cancer patient with the sulfonamide compound or the analog thereof;
(b) determining the EGFR1 expression levels in the tumor cell treated in step (a) and an untreated tumor cell; and
(c) judging that the tumor cell is highly sensitive to the sulfonamide compound or the analog thereof if the EGFR1 expression level in the tumor cell treated in step (a) is decreased compared to the EGFR1 expression level in the untreated tumor cell,

wherein the sulfonamide compound or the analog thereof is at least one compound selected from the group consisting of:

a compound represented by General Formula (I)

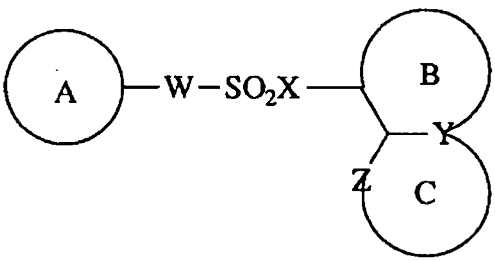

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents a carbon atom or a nitrogen atom, and

Z represents -N($R^2$)- or a nitrogen atom,

wherein, $R^1$ and $R^2$ each independently represent, identically or differently, a hydrogen atom or a lower alkyl group]; a compound represented by General Formula (II)

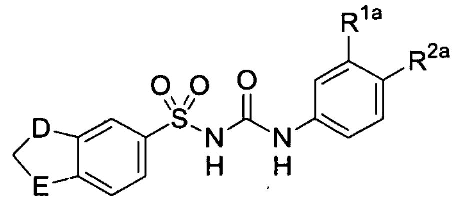

(II)

[wherein, E represents -O-, -N($CH_3$)-, -$CH_2$-, -$CH_2CH_2$- or -$CH_2O$-, D represents -$CH_2$- or -O-, $R^{1a}$ represents a hydrogen atom or a halogen atom, and $R^{2a}$ represents a halogen atom or a trifluoromethyl group];
a compound represented by General Formula (III)

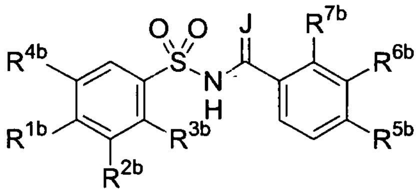

(III)

[wherein, J represents -O- or -NH-, $R^{1b}$ represents a hydrogen atom, a-halogen atom, an optionally substituted $C_1$-$C_6$ alkyl group, an optionally substituted $C_1$-$C_4$ alkoxy group, an optionally substituted $C_1$-$C_4$ alkylthio group, -$CF_3$, -$OCF_3$, -$SCF_3$, an optionally substituted $C_1$-$C_4$ alkoxy carbonyl group, a nitro group, an azido group, -O($SO_2$) $CH_3$, -N($CH_3$)$_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, -$CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, -$CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or -$CF_3$ (provided that when either one of $R^{5b}$ or $R^{7b}$ is an optionally substituted $C_1$-$C_6$ alkyl group or when $R^{7b}$ is a halogen atom or an optionally substituted $C_1$-$C_6$ alkyl group, either one of $R^{5b}$ or $R^{6b}$ is a hydrogen atom)];
a compound represented by Formula (IV)

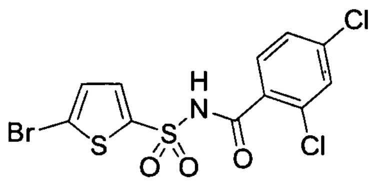

(IV)

;

and
a compound represented by Formula (V)

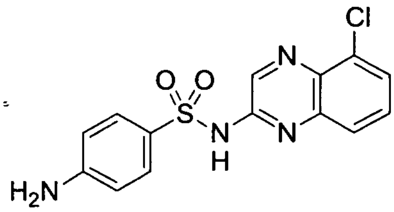 (V)

,

or a pharmacologically acceptable salt thereof, or a solvate thereof.

**3.** A method for examining the sensitivity of a tumor cell to a sulfonamide compound or an analog thereof, comprising the steps of:

(a) determining the EGFR1 expression level in a tumor cell of a cancer patient before and after the administration of the sulfonamide compound or the analog thereof; and
(b) judging that the tumor cell is highly sensitive to the sulfonamide compound or the analog thereof if the EGFR1 expression level in the tumor cell after the administration of the sulfonamide compound or the analog thereof is decreased compared to the EGFR1 expression level in the tumor cell before the administration of said compound,

wherein the sulfonamide compound or the analog thereof is at least one compound selected from the group consisting of:

a compound represented by General Formula (I)

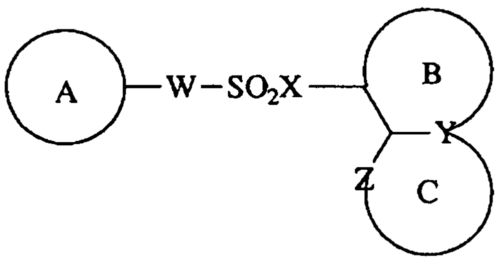 (I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents -N($R^1$)- or an oxygen atom,
Y represents a carbon atom or a nitrogen atom, and
Z represents -N($R^2$)- or a nitrogen atom,

wherein, $R^1$ and $R^2$ each independently represent, identically or differently, a hydrogen atom or a lower alkyl group];
a compound represented by General Formula (II)

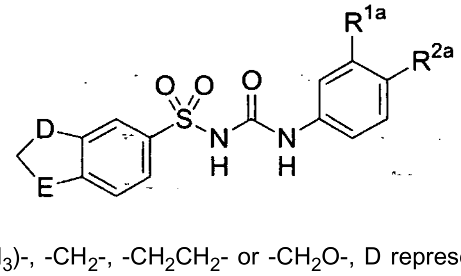 (II)

[wherein, Represent -O-, -N($CH_3$)-, -$CH_2$-, -$CH_2CH_2$- or -$CH_2O$-, D represents -$CH_2$-, or -O-, $R^{1a}$ represents a

hydrogen atom or a halogen atom, and $R^{2a}$ represents a halogen atom or a trifluoromethyl group]; a compound represented by General Formula (III)

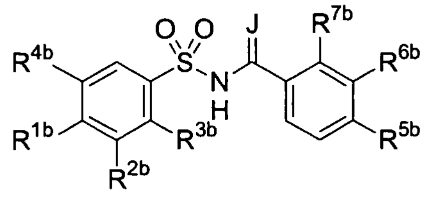

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either one of $R^{5b}$ or $R^{7b}$ is an optionally substituted $C_1$-$C_6$-alkyl group or when $R^{7b}$ is a halogen atom or an optionally substituted $C_1$-$C_6$ alkyl group, either one of $R^{5b}$ or $R^{6b}$ is a hydrogen atom)];
a compound represented by Formula (IV)

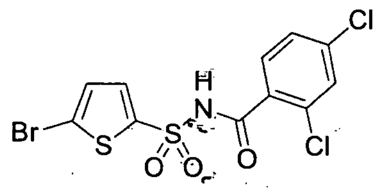

(IV)

;

and
a compound represented by Formula (V)

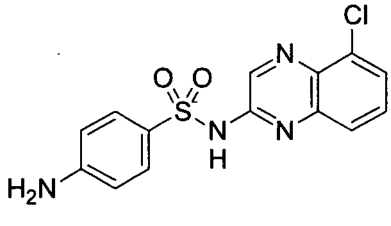

(V)

,

or a pharmacologically acceptable salt thereof, or a solvate thereof.

**4.** A method for examining the sensitivity of a tumor cell to a sulfonamide compound or an analog thereof, comprising the steps of:

(a) determining the expression level of EGFR1 in soluble form in blood drawn from a cancer patient before and after the administration of the sulfonamide compound or the analog thereof; and
(b) judging that the tumor cell is highly sensitive to the sulfonamide compound or the analog thereof if the expression level of EGFR1 in soluble form in blood drawn after the administration of the sulfonamide compound

or the analog thereof is decreased compared to the expression level of EGFR1 in soluble form in blood drawn before the administration of said compound,

wherein the sulfonamide compound or the analog thereof is at least one compound selected from the group consisting of:

a compound represented by General Formula (I)

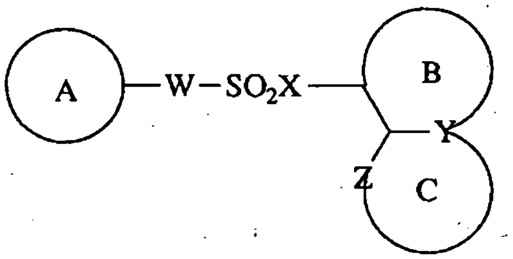

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents a carbon atom or a nitrogen atom, and
Z represents $-N(R^2)-$ or a nitrogen atom,

wherein, $R^1$ and $R^2$ each independently represent, identically or differently, a hydrogen atom or a lower alkyl group];
a compound represented by General Formula (II)

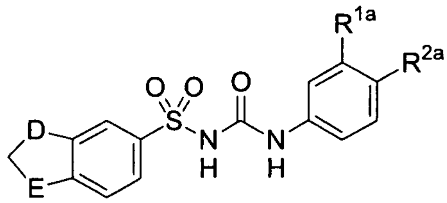

(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

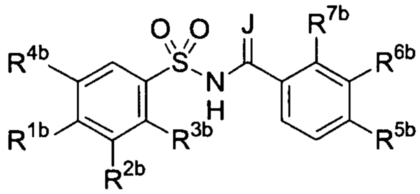

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl

group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either one of $R^{5b}$ or $R^{7b}$ is an optionally substituted $C_1$-$C_6$ alkyl group or when $R^{7b}$ is a halogen atom or an optionally substituted $C_1$-$C_6$ alkyl group, either one of $R^{5b}$ or $R^{6b}$ is a hydrogen atom)];
a compound represented by Formula (IV)

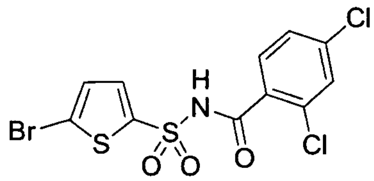
(IV)

;

and a compound represented by Formula (V)

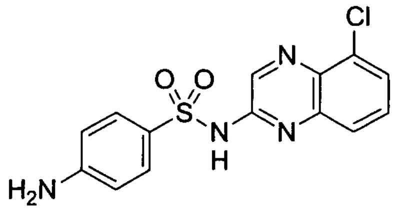
(V)

,

or a pharmacologically acceptable salt thereof, or a solvate thereof

**5.** A method for examining an anti-tumor effect of a sulfonamide compound or an analog thereof, comprising the steps of:

(a) determining the EGFR1 expression level in a tumor cell removed from a cancer patient before and after the administration of the sulfonamide compound or the analog thereof; and
(b) judging that the sulfonamide compound or the analog thereof is exerting an anti-tumor effect if the EGFR1 expression level in the tumor cell removed after the administration of the sulfonamide compound or the analog thereof is decreased compared to the EGFR1 expression level in the tumor cell removed before the administration of said compound,

wherein the sulfonamide compound or the analog thereof is at least one compound selected from the group consisting of:

a compound represented by General Formula (I)

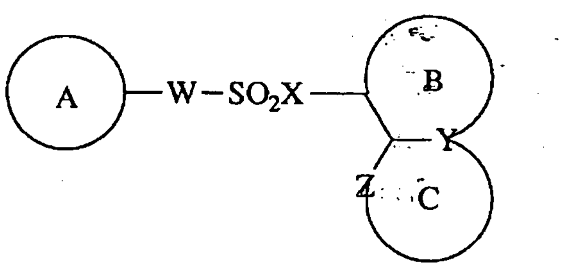
(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents $-N(R^1)-$ or an oxygen atom,
Y represents a carbon atom or a nitrogen atom, and
Z represents $-N(R^2)-$ or a nitrogen atom,

wherein, $R^1$ and $R^2$ each independently represent, identically or differently, a hydrogen atom or a lower alkyl group];
a compound represented by General Formula (II)

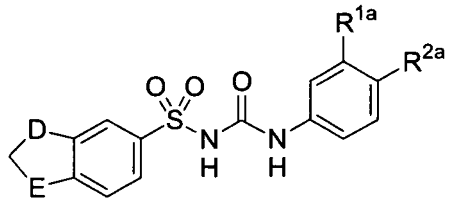

(II)

[wherein, E represents -O-, $-N(CH_3)-$, $-CH_2-$, $-CH_2CH_2-$ or $-CH_2O-$, D represents $-CH_2-$ or -O-, $R^{1a}$ represents a hydrogen atom or a halogen atom, and $R^{2a}$ represents a halogen atom or a trifluoromethyl group];
a compound represented by General Formula (III)

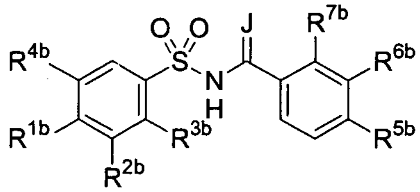

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either one of $R^{5b}$ or $R^{7b}$ is an optionally substituted $C_1$-$C_6$ alkyl group or when $R^{7b}$ is a halogen atom or an optionally substituted $C_1$-$C_6$ alkyl group, either one of $R^{5b}$ or $R^{6b}$ is a hydrogen atom)];
a compound represented by Formula (IV)

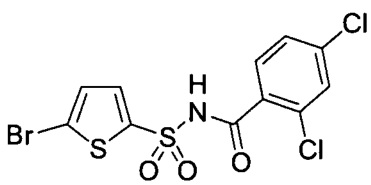

(IV)

;

and
a compound represented by Formula (V)

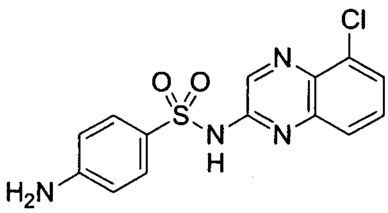

(V)

,

or a pharmacologically acceptable salt thereof, or a solvate thereof.

**6.** A method for examining an anti-tumor effect of a sulfonamide compound or an analog thereof, comprising the steps of:

(a) determining the EGFR1 expression level in a tumor cell of a cancer patient before and after the administration of the sulfonamide compound or the analog thereof; and
(b) judging that the sulfonamide compound or the analog thereof is exerting an anti-tumor effect if the EGFR1 expression level in the tumor cell after the administration of the sulfonamide compound or the analog thereof is decreased compared to the EGFR1 expression level in the tumor cell before the administration of said compound,

wherein the sulfonamide compound or the analog thereof is at least one compound selected from the group consisting of:

a compound represented by General Formula (I)

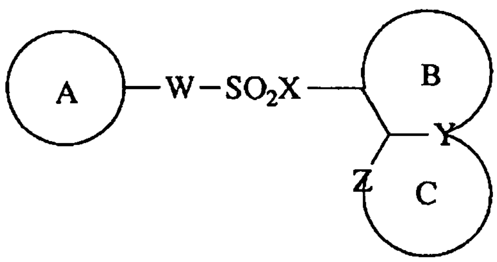

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents -N($R^1$)- or an oxygen atom,
Y represents a carbon atom or a nitrogen atom, and
Z represents -N($R^2$)- or a nitrogen atom,

wherein, $R^1$ and $R^2$ each independently represent, identically or differently, a hydrogen atom or a lower alkyl group];
a compound represented by General Formula (II)

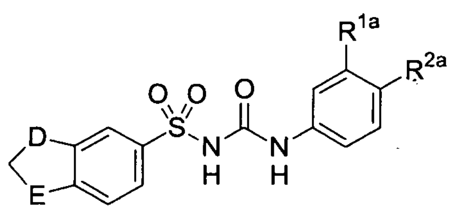

(II)

[wherein, E represents -O-, -N($CH_3$)-, -$CH_2$-, -$CH_2CH_2$- or -$CH_2O$-, D represents -$CH_2$- or -O-, $R^{1a}$ represents a

hydrogen atom or a halogen atom, and $R^{2a}$ represents a halogen atom or a trifluoromethyl group]; a compound represented by General Formula (III)

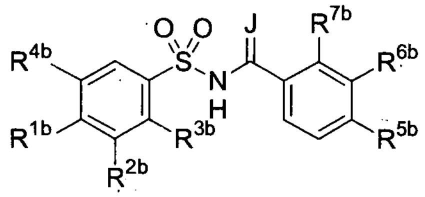

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, $-CF_3$, $-OCF_3$, $-SCF_3$, an optionally substituted $C_1$-$C_4$ alkoxy carbonyl group, a nitro group, an azido group, $-O(SO_2)CH_3$, $-N(CH_3)_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, $-CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom), $R^{5b}$ represents a hydrogen atom, a halogen atom, an optionally substituted $C_1$-$C_6$ alkyl group, $-CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, $R^{5b}$ is a hydrogen atom and $R^{7b}$ is a halogen atom), $R^{7b}$ represents a halogen atom, an optionally substituted $C_1$-$C_6$ alkyl group or $-CF_3$ (provided that when either one of $R^{5b}$ or $R^{7b}$ is an optionally substituted $C_1$-$C_6$ alkyl group or when $R^{7b}$ is a halogen atom or an optionally substituted $C_1$-$C_6$ alkyl group, either one of $R^{5b}$ or $R^{6b}$ is a hydrogen atom)];
a compound represented by Formula (IV)

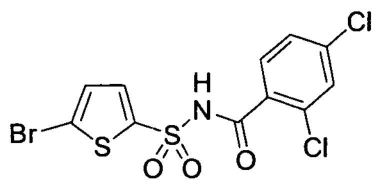

(IV)

;

and
a compound represented by Formula (V)

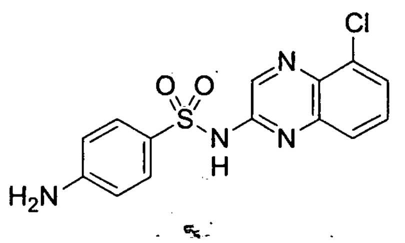

(V)

,

or a pharmacologically acceptable salt thereof, or a solvate thereof.

**7.** A method for examining an anti-tumor effect of a sulfonamide compound or an analog thereof, comprising the steps of:

(a) determining the expression level of EGFR1 in soluble form in blood drawn from a cancer patient before and after the administration of the sulfonamide compound or the analog thereof; and
(b) judging that the sulfonamide compound or the analog thereof is exerting an anti-tumor effect if the expression level of EGFR1 in soluble form in blood drawn after the administration of the sulfonamide compound or the analog thereof is decreased compared to the expression level of EGFR1 in soluble form in blood drawn before the administration of said compound,

wherein the sulfonamide compound or the analog thereof is at least one compound selected from the group consisting of:

a compound represented by General Formula (I)

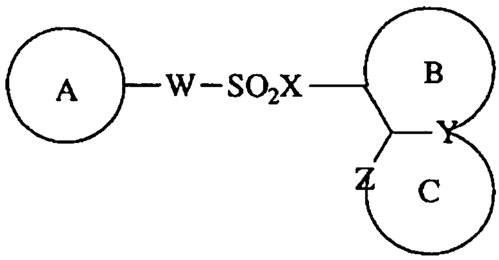

(I)

[wherein, ring A represents an optionally substituted monocyclic or bicyclic aromatic ring,
ring B represents an optionally substituted 6-membered cyclic unsaturated hydrocarbon or 6-membered unsaturated heterocycle containing a nitrogen atom as a heteroatom,
ring C represents an optionally substituted 5-membered heterocycle containing one or two nitrogen atoms,
W represents a single bond or -CH=CH-,
X represents -N($R^1$)- or an oxygen atom,
Y represents a carbon atom or a nitrogen atom, and
Z represents -N($R^2$)- or a nitrogen atom,

wherein, $R^1$ and $R^2$ each independently represent, identically or differently, a hydrogen atom or a lower alkyl group];
a compound represented by General Formula (II)

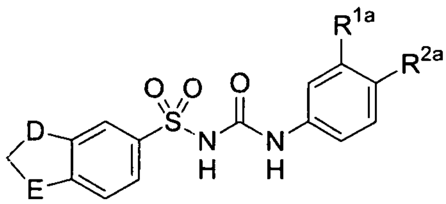

(II)

[wherein, E represents -O-, -N($CH_3$)-, -$CH_2$-, -$CH_2CH_2$- or -$CH_2O$-, D represents -$CH_2$- or -O-, $R^{1a}$ represents a hydrogen atom or a halogen atom, and $R^{2a}$ represents a halogen atom or a trifluoromethyl group];
a compound represented by General Formula (III)

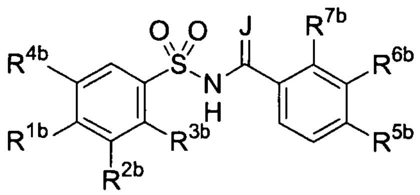

(III)

[wherein, J represents -O- or -NH-, $R^{1b}$ represents a hydrogen atom, a halogen atom, an optionally substituted $C_1$-$C_6$ alkyl group, an optionally substituted $C_1$-$C_4$ alkoxy group, an optionally substituted $C_1$-$C_4$ alkylthio group, -$CF_3$, -$OCF_3$, -$SCF_3$, an optionally substituted $C_1$-$C_4$ alkoxy carbonyl group, a nitro group, an azido group, -O($SO_2$)$CH_3$, -N($CH_3$)$_2$, a hydroxyl group, a phenyl group, a substituted phenyl group, a pyridinyl group, a thienyl group, a furyl group, a quinolinyl group or a triazole group, $R^{2b}$ represents a hydrogen atom, a halogen atom, a cyano group, -$CF_3$, an optionally substituted $C_1$-$C_6$ alkyl group, an optionally substituted $C_1$-$C_4$ alkoxy carbonyl group, an optionally substituted $C_1$-$C_4$ alkoxy group, an optionally substituted phenyl group or an optionally substituted quinolinyl group, $R^{3b}$ represents a hydrogen atom or an optionally substituted $C_1$-$C_4$ alkoxy group, $R^{4b}$ represents a hydrogen atom or an optionally substituted $C_1$-$C_6$ alkyl group (provided that at least one of $R^{3b}$ and $R^{4b}$ is a hydrogen atom),

$R^{5b}$ represents a hydrogen atom, a halogen atom, an optionally substituted $C_1$-$C_6$ alkyl group, -$CF_3$ or a nitro group, $R^{6b}$ represents a hydrogen atom, a halogen atom or an optionally substituted $C_1$-$C_6$ alkyl group (provided that when $R^{6b}$ is an optionally substituted $C_1$-$C_6$ alkyl group, , is a hydrogen atom and $R^{7b}$ is a halogen atom), $R^{7b}$ represents a halogen atom, an optionally substituted $C_1$-$C_6$ alkyl group or -$CF_3$ (provided that when either one of $R^{5b}$ or $R^{7b}$ is an optionally substituted $C_1$-$C_6$ alkyl group or when $R^{7b}$ is a halogen atom or an optionally substituted $C_1$-$C_6$ alkyl group, either one of $R^{5b}$ or $R^{6b}$ is a hydrogen atom)];
a compound represented by Formula (IV)

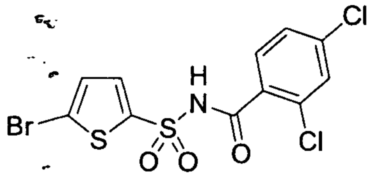

(IV)

;

and
a compound represented by Formula (V)

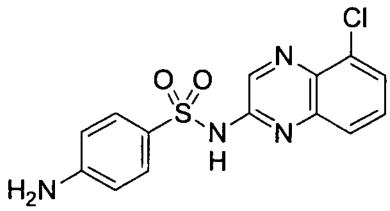

(V)

,

or a pharmacologically acceptable salt thereof, or a solvate thereof.

**8.** A method according to any one of Claims 1 to 7 above, wherein the sulfonamide compound is at least one compound selected from the group consisting of:

N-(3-chloro-1H-indole-7-yl)-1,4-benzenedisulfonamide
N-(3-cyano-4-methyl-1H-indole-7-yl)-3-cyanobenzenesulfonamide,
N-[[(4-chlorophenyl)amino]carbonyl]-2,3-dihydro-1H-indene-5-sulfonamide,
N-[[(3,4-dichlorophenyl)amino]carbonyl]-2,3-dihydrobenzofuran-5-sulfonamide,
N-(2,4-dichlorobenzoyl)-4-chlorophenylsulfonamide,
N-(2,4-dichlorobenzoyl)-5-bromothiophene-2-sulfonamide and
2-sulfanylamide-5-chloroquinoxaline,

a pharmacologically acceptable salt thereof or a solvate thereof.

**9.** A method according to any one of Claims 1 to 7, wherein the sulfonamide compound is N-(3-chloro-1H-indole-7-yl)-1,4-benzenedisulfonamide, a pharmacologically acceptable salt thereof or a solvate thereof.

**10.** A method according to any one of Claims 1 to 7, wherein the sulfonamide compound is N-(3-cyano-4-methyl-1H-indole-7-yl)-3-cyanobenzenesulfonamide, a pharmacologically acceptable salt thereof or a solvate thereof.

**11.** A method according to any one of Claims 1 to 7, wherein the sulfonamide compound is at least one compound selected from the group consisting of N-[[(3,4-dichlorophenyl)amino]carbonyl]-2,3-dihydrobenzofuran-5-sulfonamide and N-(2,4-dichlorobenzoyl)-5-bromothiophene-2-sulfonamide, a pharmacologically acceptable salt thereof or a solvate thereof.

**12.** A method according to any one of Claims 1 to 7, wherein the sulfonamide compound is N-(2,4-dichlorobenzoyl)-5-bromothiophene-2-sulfonamide in sodium salt form.

**13.** A method according to any one of Claims 1 to 12, wherein the determination of the EGFR1 expression level is carried out by quantifying protein of EGFR1.

**14.** A method according to Claims 13, wherein the quantification of protein is carried out by at least one selected from the group consisting of immunochemical technique, mass spectroscopy, SPECT and PET.

**15.** A method according to Claim 14, wherein the immunochemical technique is at least one technique selected from the group consisting of immunohistochemical technique, flow cytometry and western blotting.

**16.** A test kit used with the method according to Claim 13, comprising an antibody having affinity for EGFR1.

**17.** A method according to any one of Claims 1 to 12, wherein the determination of the EGFR1 expression level is carried out by quantifying the phosphorylation level of EGFR1.

**18.** A method according to Claim 17, wherein the quantification of the phosphorylation level of EGFR1 is carried out by at least one selected from the group consisting of immunochemical technique, mass spectroscopy, SPECT and PET.

**19.** A method according to Claim 18, wherein the immunochemical technique is at least one technique selected from the group consisting of immunohistochemical technique, flow cytometry and western blotting.

**20.** A test kit used with the method according to Claim 17, comprising an antibody having affinity for phosphorylated EGFR1.

# Figure 1

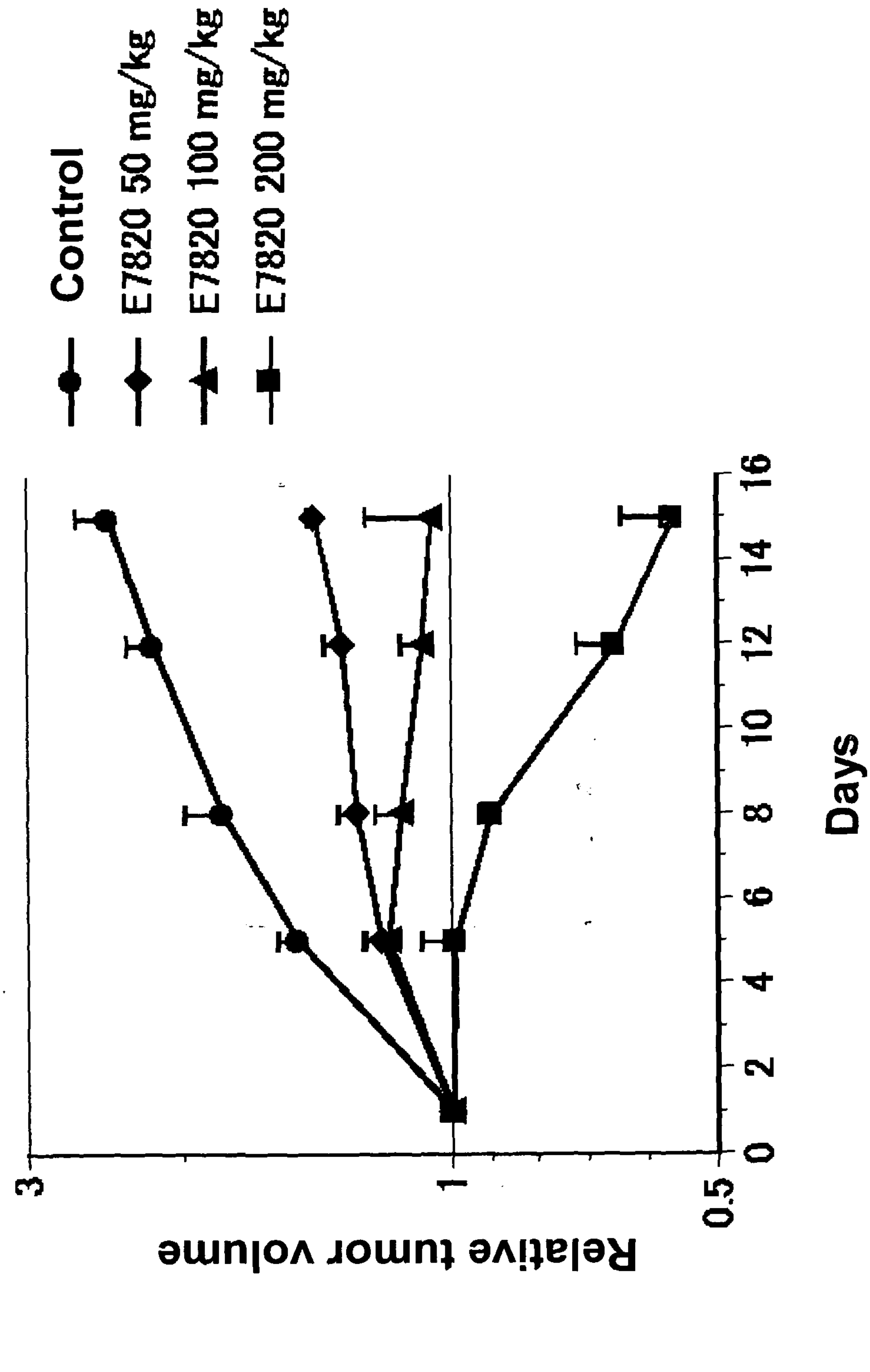
Control
E7820 50 mg/kg
E7820 100 mg/kg
E7820 200 mg/kg
Relative tumor volume
3
1
0.5
0
2
4
6
8
10
12
14
16
Days

Figure 2

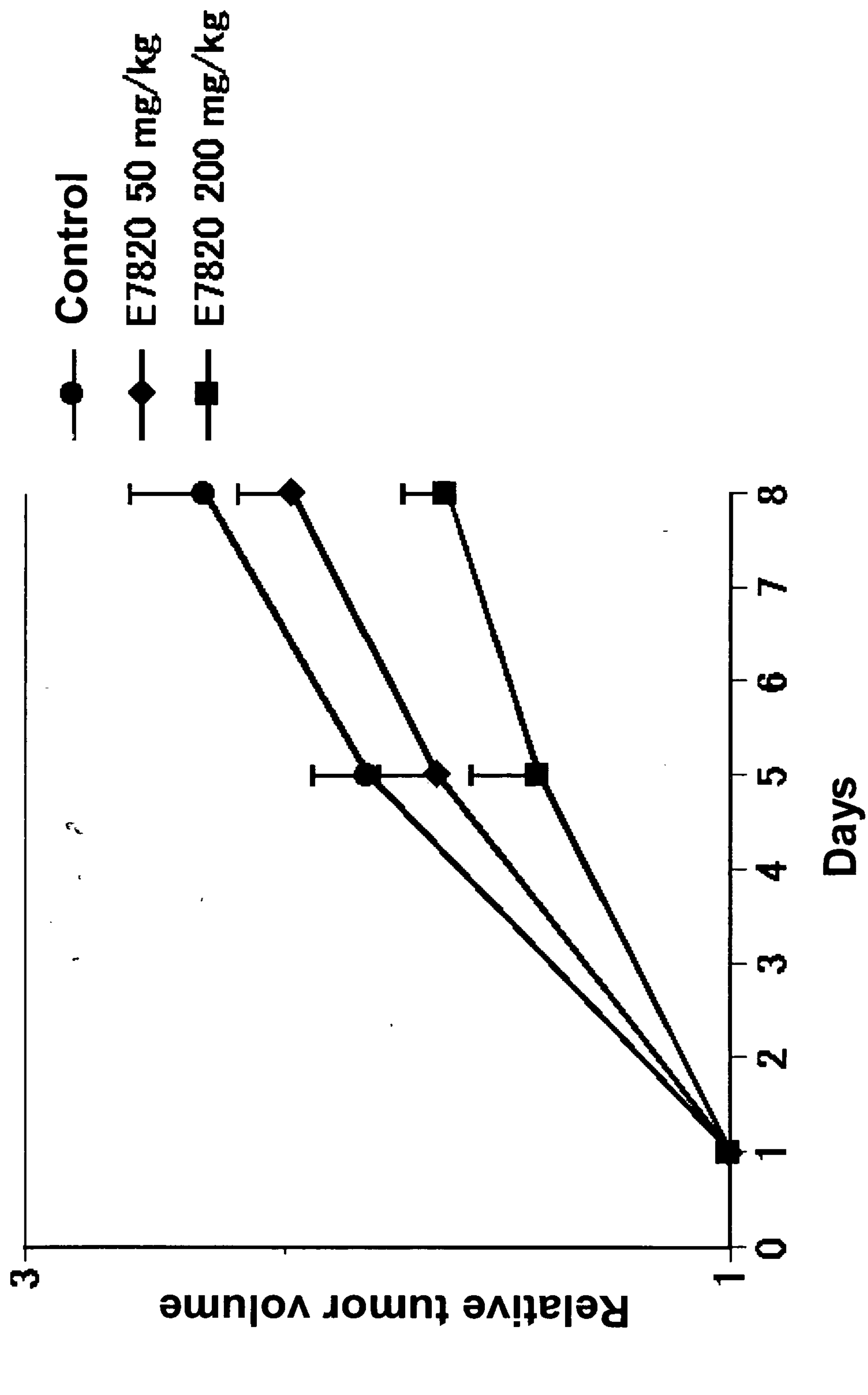
Control
E7820 50 mg/kg
E7820 200 mg/kg
Relative tumor volume
3
1
Days
0
1
2
3
4
5
6
7
8

# Figure 3

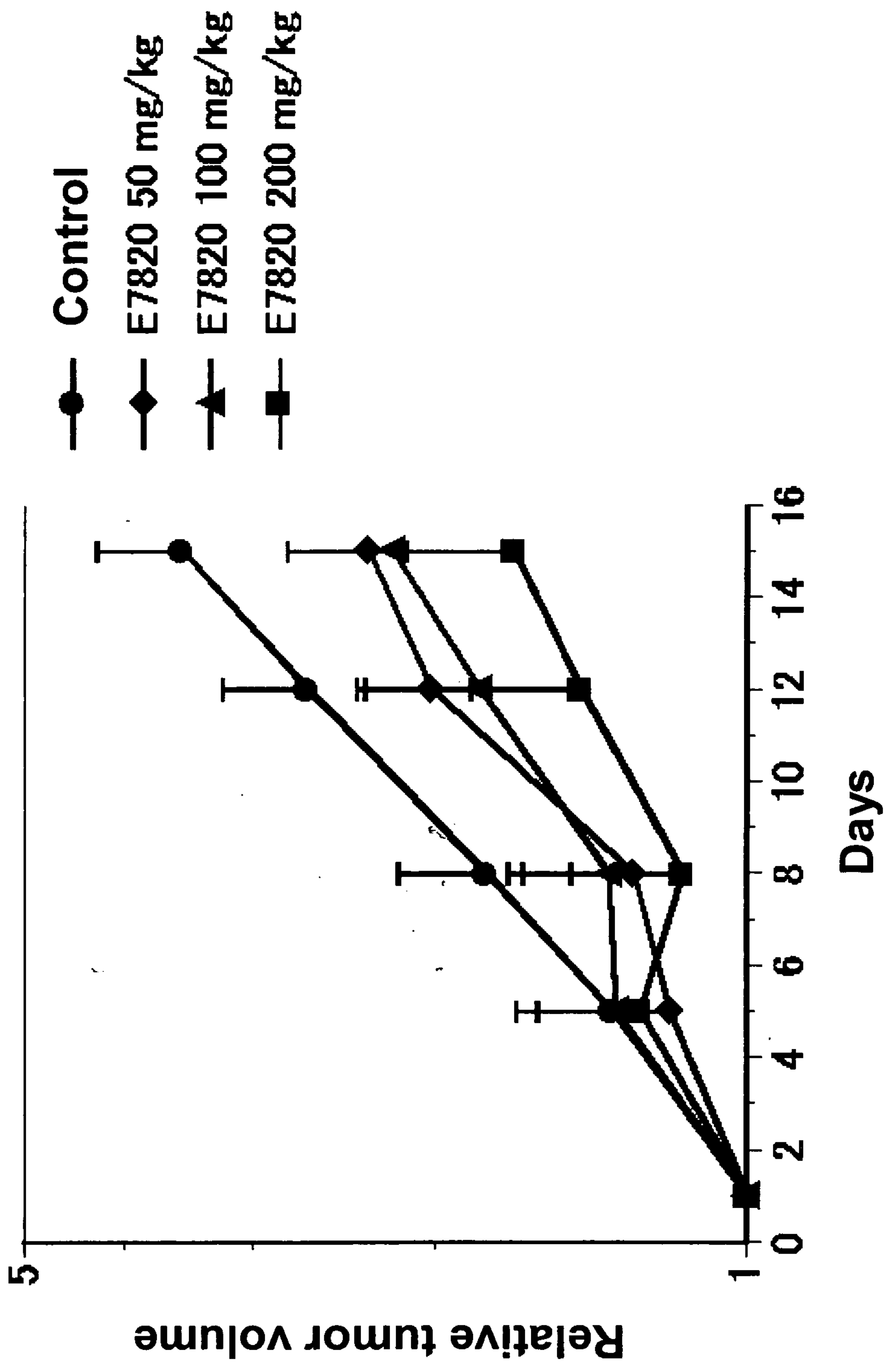
Control
E7820 50 mg/kg
E7820 100 mg/kg
E7820 200 mg/kg
Relative tumor volume
5
1
0
2
4
6
8
10
12
14
16
Days

# Figure 4

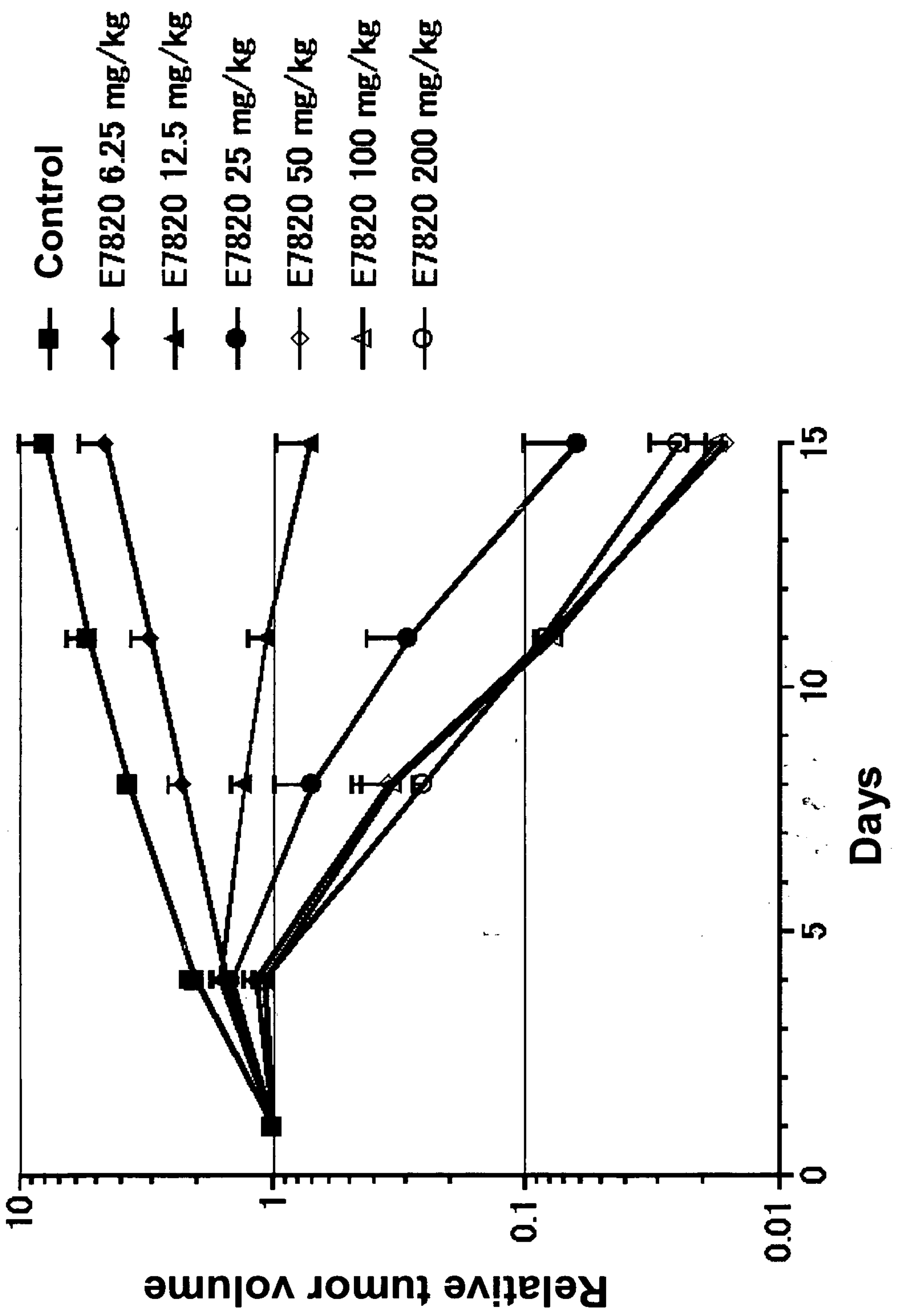
Control
E7820 6.25 mg/kg
E7820 12.5 mg/kg
E7820 25 mg/kg
E7820 50 mg/kg
E7820 100 mg/kg
E7820 200 mg/kg
Relative tumor volume
10
1
0.1
0.01
Days
0
5
10
15

# Figure 5

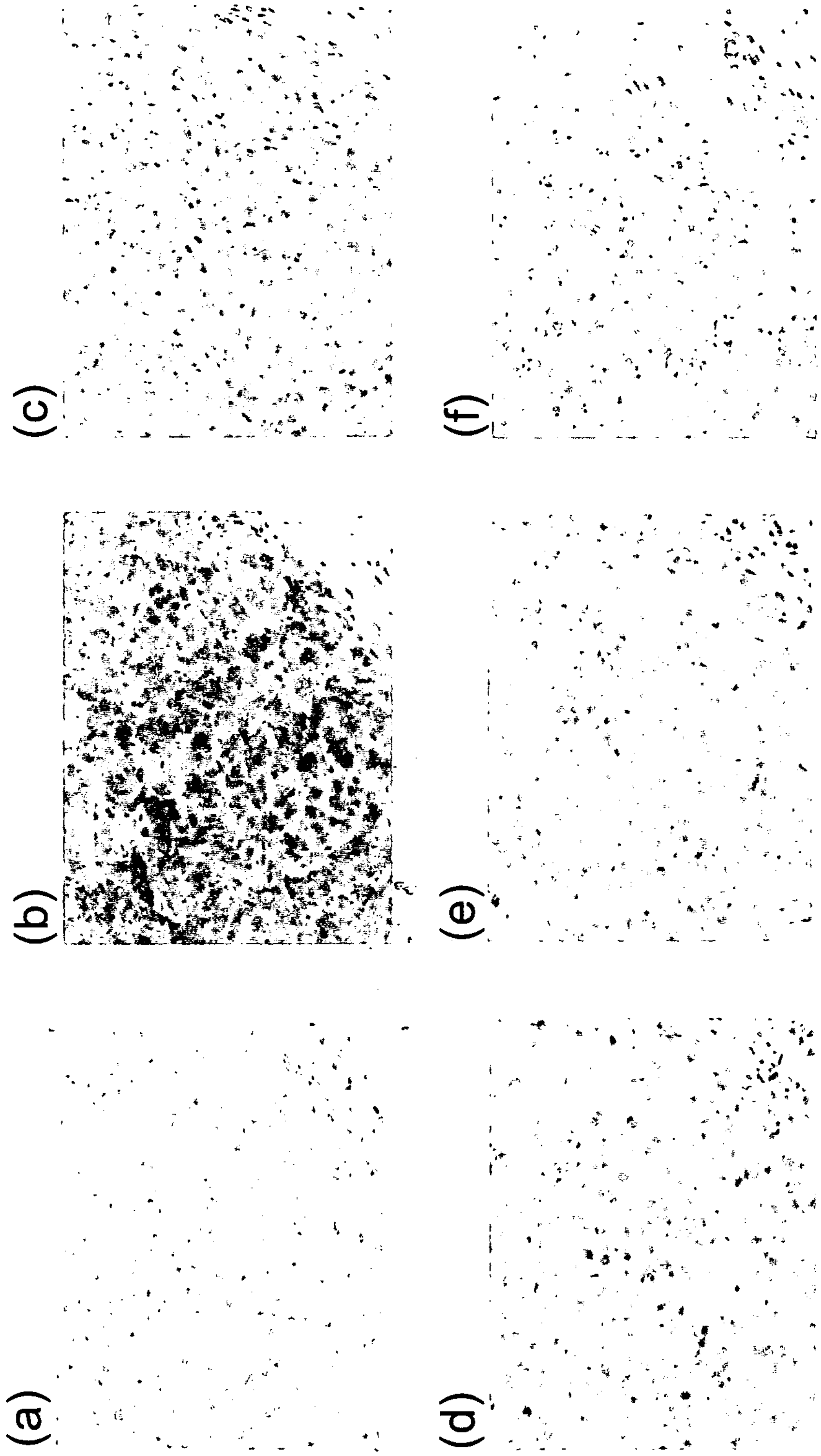

**INTERNATIONAL SEARCH REPORT**

International application No. PCT/JP2007/059139

A. CLASSIFICATION OF SUBJECT MATTER
*G01N33/53*(2006.01)i, *A61K31/404*(2006.01)i, *A61P35/00*(2006.01)i, *C12Q1/02*(2006.01)i, *G01N33/15*(2006.01)i

According to International Patent Classification (IPC) or to both national classification and IPC

B. FIELDS SEARCHED

Minimum documentation searched (classification system followed by classification symbols)
G01N33/53, A61K31/404, A61P35/00, C12Q1/02, G01N33/15

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CA(STN), EMBASE(STN), MEDLINE(STN), WPIDS(STN), JMEDPlus(JDream2), JST7580(JDream2),

C. DOCUMENTS CONSIDERED TO BE RELEVANT

| Category* | Citation of document, with indication, where appropriate, of the relevant passages | Relevant to claim No. |
|---|---|---|
| Y | WO 2006/036025 A1 (Eisai Co., Ltd.), 06 April, 2006 (06.04.06), Full text (Family: none) | 1-20 |
| Y | JP 2004-529315 A (Board of Regents, the University of Texas System), 24 September, 2004 (24.09.04), Full text & US 2002/0132275 A1 & EP 1332368 A & WO 2002/039121 A2 & AU 3657202 A & CA 2427622 A | 1-20 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 May, 2007 (24.05.07) | 05 June, 2007 (05.06.07) |
| Name and mailing address of the ISA/ Japanese Patent Office | Authorized officer |
| Facsimile No. | Telephone No. |

Form PCT/ISA/210 (second sheet) (April 2005)

INTERNATIONAL SEARCH REPORT

International application No.
PCT/JP2007/059139

Continuation of B. FIELDS SEARCHED
Electronic data base consulted during the international search (name of data base and, where practicable, search terms used)

JSTPlus(JDream2) EPIDERMAL(W)GROWTH(W)FACTOR(W) RECEPTOR(W)1, CANCER, CARCINOMA, TUMOR

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0242493 A
- WO 2006036025 A
- JP 7165708 A
- WO 0050395 A
- EP 0222475 A
- WO 02098848 A
- WO 03035629 A
- WO 2006030941 A
- WO 2006030947 A
- WO 02042493 A
- JP 2006117183 A
- WO 9507276 A
- EP 0222475 A1
- EP 0555036 A2
- WO 2003035629 A
- WO 2005050188 A


### Non-patent literature cited in the description


- *Molecular Cancer Therapeutics,* 2002, vol. 1, 275-286
- *J. Am. Chem. Soc.,* 1947, vol. 71, 6-10
- *Current Opinion in Cell Biology,* 1999, vol. 11, 184-189
- *Nature Genetics,* 2005, vol. 37, 1315-1316
- *Human Pathology,* 2005, vol. 36, 1127-1134
- **HAMBURGER et al.** *Cancer Research,* 1978, vol. 38, 3438-3443
- *Science,* 1977, vol. 197, 461-463
- *Journal of Clinical Investigation,* 1977, vol. 60, 846-854
- *Proceeding, American. Association. Cancer Research,* 1979, vol. 20, 51
- **A.T.BARON et al.** *J. Immunol. Methods,* 1998, vol. 219, 23-43

SEQUENCE LISTING

<110> Eisai R&D Management Co., Ltd.

<120> Novel marker for sensitivity against sulfonamide compound

<130> G08-0051

<140> PCT/JP2007/059139
<141> 2007-04-20

<150> JP 2006-117183
<151> 2006-04-20

<160> 1

<170> PatentIn version 3.3

<210> 1
<211> 1210
<212> PRT
<213> Homo sapiens

<400> 1

Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
        35                  40                  45

Leu Ser Leu Gln Arg Met Phe Asn Asn Cys Glu Val Val Leu Gly Asn
    50                  55                  60

Leu Glu Ile Thr Tyr Val Gln Arg Asn Tyr Asp Leu Ser Phe Leu Lys
65                  70                  75                  80

Thr Ile Gln Glu Val Ala Gly Tyr Val Leu Ile Ala Leu Asn Thr Val
85 90 95

Glu Arg Ile Pro Leu Glu Asn Leu Gln Ile Ile Arg Gly Asn Met Tyr
100 105 110

Tyr Glu Asn Ser Tyr Ala Leu Ala Val Leu Ser Asn Tyr Asp Ala Asn
115 120 125

Lys Thr Gly Leu Lys Glu Leu Pro Met Arg Asn Leu Gln Glu Ile Leu
130 135 140

His Gly Ala Val Arg Phe Ser Asn Asn Pro Ala Leu Cys Asn Val Glu
145 150 155 160

Ser Ile Gln Trp Arg Asp Ile Val Ser Ser Asp Phe Leu Ser Asn Met
165 170 175

Ser Met Asp Phe Gln Asn His Leu Gly Ser Cys Gln Lys Cys Asp Pro
180 185 190

Ser Cys Pro Asn Gly Ser Cys Trp Gly Ala Gly Glu Glu Asn Cys Gln
195 200 205

Lys Leu Thr Lys Ile Ile Cys Ala Gln Gln Cys Ser Gly Arg Cys Arg
210 215 220

Gly Lys Ser Pro Ser Asp Cys Cys His Asn Gln Cys Ala Ala Gly Cys
225 230 235 240

Thr Gly Pro Arg Glu Ser Asp Cys Leu Val Cys Arg Lys Phe Arg Asp
245 250 255

Glu Ala Thr Cys Lys Asp Thr Cys Pro Pro Leu Met Leu Tyr Asn Pro
260 265 270

Thr Thr Tyr Gln Met Asp Val Asn Pro Glu Gly Lys Tyr Ser Phe Gly
275 280 285

Ala Thr Cys Val Lys Lys Cys Pro Arg Asn Tyr Val Val Thr Asp His
290 295 300

Gly Ser Cys Val Arg Ala Cys Gly Ala Asp Ser Tyr Glu Met Glu Glu
305 310 315 320

Asp Gly Val Arg Lys Cys Lys Lys Cys Glu Gly Pro Cys Arg Lys Val
325 330 335

Cys Asn Gly Ile Gly Ile Gly Glu Phe Lys Asp Ser Leu Ser Ile Asn
340 345 350

Ala Thr Asn Ile Lys His Phe Lys Asn Cys Thr Ser Ile Ser Gly Asp
355 360 365

Leu His Ile Leu Pro Val Ala Phe Arg Gly Asp Ser Phe Thr His Thr
370 375 380

Pro Pro Leu Asp Pro Gln Glu Leu Asp Ile Leu Lys Thr Val Lys Glu
385 390 395 400

Ile Thr Gly Phe Leu Leu Ile Gln Ala Trp Pro Glu Asn Arg Thr Asp
405 410 415

Leu His Ala Phe Glu Asn Leu Glu Ile Ile Arg Gly Arg Thr Lys Gln
420 425 430

His Gly Gln Phe Ser Leu Ala Val Val Ser Leu Asn Ile Thr Ser Leu
435 440 445

Gly Leu Arg Ser Leu Lys Glu Ile Ser Asp Gly Asp Val Ile Ile Ser
450 455 460

Gly Asn Lys Asn Leu Cys Tyr Ala Asn Thr Ile Asn Trp Lys Lys Leu
465 470 475 480

Phe Gly Thr Ser Gly Gln Lys Thr Lys Ile Ile Ser Asn Arg Gly Glu
485 490 495

Asn Ser Cys Lys Ala Thr Gly Gln Val Cys His Ala Leu Cys Ser Pro
500 505 510

Glu Gly Cys Trp Gly Pro Glu Pro Arg Asp Cys Val Ser Cys Arg Asn
515 520 525

Val Ser Arg Gly Arg Glu Cys Val Asp Lys Cys Asn Leu Leu Glu Gly
530 535 540

Glu Pro Arg Glu Phe Val Glu Asn Ser Glu Cys Ile Gln Cys His Pro
545 550 555 560

Glu Cys Leu Pro Gln Ala Met Asn Ile Thr Cys Thr Gly Arg Gly Pro
565 570 575

Asp Asn Cys Ile Gln Cys Ala His Tyr Ile Asp Gly Pro His Cys Val
580 585 590

Lys Thr Cys Pro Ala Gly Val Met Gly Glu Asn Asn Thr Leu Val Trp
595 600 605

Lys Tyr Ala Asp Ala Gly His Val Cys His Leu Cys His Pro Asn Cys
    610                 615                 620

Thr Tyr Gly Cys Thr Gly Pro Gly Leu Glu Gly Cys Pro Thr Asn Gly
625                 630                 635                 640

Pro Lys Ile Pro Ser Ile Ala Thr Gly Met Val Gly Ala Leu Leu Leu
                645                 650                 655

Leu Leu Val Val Ala Leu Gly Ile Gly Leu Phe Met Arg Arg Arg His
            660                 665                 670

Ile Val Arg Lys Arg Thr Leu Arg Arg Leu Leu Gln Glu Arg Glu Leu
        675                 680                 685

Val Glu Pro Leu Thr Pro Ser Gly Glu Ala Pro Asn Gln Ala Leu Leu
    690                 695                 700

Arg Ile Leu Lys Glu Thr Glu Phe Lys Lys Ile Lys Val Leu Gly Ser
705                 710                 715                 720

Gly Ala Phe Gly Thr Val Tyr Lys Gly Leu Trp Ile Pro Glu Gly Glu
                725                 730                 735

Lys Val Lys Ile Pro Val Ala Ile Lys Glu Leu Arg Glu Ala Thr Ser
            740                 745                 750

Pro Lys Ala Asn Lys Glu Ile Leu Asp Glu Ala Tyr Val Met Ala Ser
        755                 760                 765

Val Asp Asn Pro His Val Cys Arg Leu Leu Gly Ile Cys Leu Thr Ser
    770                 775                 780

Thr Val Gln Leu Ile Thr Gln Leu Met Pro Phe Gly Cys Leu Leu Asp
785 790 795 800

Tyr Val Arg Glu His Lys Asp Asn Ile Gly Ser Gln Tyr Leu Leu Asn
805 810 815

Trp Cys Val Gln Ile Ala Lys Gly Met Asn Tyr Leu Glu Asp Arg Arg
820 825 830

Leu Val His Arg Asp Leu Ala Ala Arg Asn Val Leu Val Lys Thr Pro
835 840 845

Gln His Val Lys Ile Thr Asp Phe Gly Leu Ala Lys Leu Leu Gly Ala
850 855 860

Glu Glu Lys Glu Tyr His Ala Glu Gly Gly Lys Val Pro Ile Lys Trp
865 870 875 880

Met Ala Leu Glu Ser Ile Leu His Arg Ile Tyr Thr His Gln Ser Asp
885 890 895

Val Trp Ser Tyr Gly Val Thr Val Trp Glu Leu Met Thr Phe Gly Ser
900 905 910

Lys Pro Tyr Asp Gly Ile Pro Ala Ser Glu Ile Ser Ser Ile Leu Glu
915 920 925

Lys Gly Glu Arg Leu Pro Gln Pro Pro Ile Cys Thr Ile Asp Val Tyr
930 935 940

Met Ile Met Val Lys Cys Trp Met Ile Asp Ala Asp Ser Arg Pro Lys
945 950 955 960

Phe Arg Glu Leu Ile Ile Glu Phe Ser Lys Met Ala Arg Asp Pro Gln
965 970 975

Arg Tyr Leu Val Ile Gln Gly Asp Glu Arg Met His Leu Pro Ser Pro
980 985 990

Thr Asp Ser Asn Phe Tyr Arg Ala Leu Met Asp Glu Glu Asp Met Asp
995 1000 1005

Asp Val Val Asp Ala Asp Glu Tyr Leu Ile Pro Gln Gln Gly Phe
1010 1015 1020

Phe Ser Ser Pro Ser Thr Ser Arg Thr Pro Leu Leu Ser Ser Leu
1025 1030 1035

Ser Ala Thr Ser Asn Asn Ser Thr Val Ala Cys Ile Asp Arg Asn
1040 1045 1050

Gly Leu Gln Ser Cys Pro Ile Lys Glu Asp Ser Phe Leu Gln Arg
1055 1060 1065

Tyr Ser Ser Asp Pro Thr Gly Ala Leu Thr Glu Asp Ser Ile Asp
1070 1075 1080

Asp Thr Phe Leu Pro Val Pro Glu Tyr Ile Asn Gln Ser Val Pro
1085 1090 1095

Lys Arg Pro Ala Gly Ser Val Gln Asn Pro Val Tyr His Asn Gln
1100 1105 1110

Pro Leu Asn Pro Ala Pro Ser Arg Asp Pro His Tyr Gln Asp Pro
1115 1120 1125

```
His Ser Thr Ala Val Gly Asn Pro Glu Tyr Leu Asn Thr Val Gln
    1130                    1135                1140

Pro Thr Cys Val Asn Ser Thr Phe Asp Ser Pro Ala His Trp Ala
    1145                    1150                1155

Gln Lys Gly Ser His Gln Ile Ser Leu Asp Asn Pro Asp Tyr Gln
    1160                    1165                1170

Gln Asp Phe Phe Pro Lys Glu Ala Lys Pro Asn Gly Ile Phe Lys
    1175                    1180                1185

Gly Ser Thr Ala Glu Asn Ala Glu Tyr Leu Arg Val Ala Pro Gln
    1190                    1195                1200

Ser Ser Glu Phe Ile Gly Ala
    1205                    1210
```